# EUROPÄISCHE PATENTANMELDUNG

(11) **EP 3 107 019 A1**
(43) Veröffentlichungstag der Anmeldung: **21.12.2016**
(21) Anmeldenummer: 15001768.9
(22) Anmeldetag: 16.06.2015
(51) Int. Cl.: G06F 19/00

(54) **VERFAHREN, COMPUTERPROGRAMMPRODUKT UND SYSTEM ZUM AUSTAUSCH VON GESUNDHEITSDATEN**

(71) Anmelder: Lazaryev, Oleksiy, 50344 Kaunas (LT)
(72) Erfinder: Oleksiy, Lazaryev, LT 50344 Kaunas (LT); Platon, Lazaryev, 04208 Kiev (UA); Maryan, Karpyk, 85586 Poing (DE)
(74) Vertreter: Tesch-Biedermann, Carmen

(57) **Zusammenfassung**

Offenbart werden ein Verfahren zum Austausch von Gesundheitsdaten zwischen einem Computersystem und einem Nutzer, ein Computerprogrammprodukt zum Ausführen des Verfahrens sowie ein zum Ausführen des Verfahrens geeignetes System. Das Computersystem verfügt dabei über eine spezielle Nutzerdatenbank sowie über eine spezielle medizinische Datenbank, in der Nutzerklassen-spezifische Gesundheitsdaten gespeichert sind. Durch die vom System angestoßene Kommunikation mit einem Nutzer gemäß einem näher definierten Kommunikationsprotokoll werden Gesundheitsdaten erhalten, die umfangreich genutzt werden können.

## Beschreibung

### Gebiet der Erfindung

Die Erfindung betrifft ein Verfahren zum Austausch von Gesundheitsdaten zwischen einem Computersystem und einem Nutzer sowie ein Computerprogrammprodukt mit einem Programmcode zum Ausführen des Verfahrens. Des Weiteren betrifft die Erfindung ein System zum Austausch von Gesundheitsdaten zwischen einem Server und einem Nutzer.

### Stand der Technik

Aus dem Stand der Technik sind bereits diverse Anwendungen der Informationstechnologie (IT) in der Medizin und in der Medizintechnik bekannt. In der medizinischen Forschung wird beispielsweise umfangreiches Datenmaterial aus Studien in speziellen Datenbanken abgelegt, verwaltet und analysiert (vgl. zum Beispiel EP 1 521 200 A2). In der Medizintechnik werden medizinische Geräte vermehrt computergesteuert und in Computersysteme integriert (vgl. zum Beispiel EP 1 883 197 B1). Für einen Patienten bzw. für einen Krankenversicherten war sicherlich die Einführung der Gesundheitskarte eine deutlich wahrnehmbare Veränderung. Dennoch existieren die bereits bekannten IT-Anwendungen noch relativ isoliert voneinander. Insbesondere der Patient bzw. Nutzer wird noch ungenügend in die existierenden Systeme und Verfahren integriert.

### Beschreibung der Erfindung

Es ist deshalb die Aufgabe der vorliegenden Erfindung, ein verbessertes Verfahren sowie ein verbessertes System zum Austausch von Gesundheitsdaten zwischen einem Computersystem und einem Nutzer bereitzustellen.

Die Aufgabe wird gelöst durch den Gegenstand der unabhängigen Patentansprüche. Vorteilhafte Ausführungsformen der Erfindung gehen aus den abhängigen Patentansprüchen hervor.

Das erfindungsgemäße Verfahren und System zum Austausch von Gesundheitsdaten bzw. Gesundheitsinformation zwischen einem Nutzer und einem Computersystem bzw. Server und ggf. anderen Teilnehmern des Datenaustausches arbeitet nach einem speziellen Kommunikationsprotokoll und weist diverse Vorteile auf. Die Erfindung erlaubt einen verbesserten Datenaustausch zwischen verschiedenen Akteuren des Gesundheitswesens, das den eigentlich Betroffenen, nämlich den Nutzer bzw. Patienten, explizit miteinschließt. Ein verbesserter Datenaustausch sorgt dafür, dass einem behandelnden Arzt oder medizinischem Versorgungszentrum tatsächlich alle Informationen medizinischer Art über einen Patienten bereitstehen, was eine effizientere Behandlung, Bekämpfung von Krankheiten und bessere Behandlungserfolge garantiert. Gerade in besonders kritischen Krankheitssituationen ist der umfassende Informationsaustausch zwischen allen behandelnden Ärzten ein kritischer Faktor, der über Heilung, Verhinderung von Komplikationen, Verbesserung der Gesundheit oder gegebenenfalls Tod eines Patienten entscheidet.

Außerdem erlaubt die Erfindung eine verbesserte Dokumentation und Erfassung von Gesundheitsdaten. Diese kann sogar lückenlos sein, so dass ein Arzt unter Rückgriff auf diese Daten bessere Therapieansätze für einen Patienten auswählen und bessere Behandlungserfolge erzielen kann.

Schließlich stellt die Erfindung eine Möglichkeit bereit, wie ein Patient bzw. Nutzer seine Gesundheitswerte selbst monitoren bzw. selbst kontrollieren kann. Im Falle von Problemen oder einer etwaigen Verschlechterung von Werten kann so früher und zielgerichteter ein Arzt aufgesucht werden. Insgesamt erfolgt eine Sensibilisierung des Nutzers für seinen Gesundheitszustand. Eine entsprechende Sensibilisierung hat aber eine gesundheitsbewusstere Lebensweise zur Folge, so dass der Gesundheitszustand eines Nutzers des Systems nachhaltig verbessert werden kann. Ein Patient, der über seinen aktuellen Gesundheitszustand informiert ist und Gesundheitsempfehlungen umsetzt, kann ein motivierendes Feedback über das erfindungsgemäße Verfahren bzw. System erhalten. Im Rahmen dieses Feedbacks kann ein Belohnungssystem implementiert werden (Scoring), das einem Patienten bzw. Nutzer der Erfindung handfeste Vorteile wie zum Beispiel Prämienvorteile bei Versicherungen etc. verschaffen kann.

Zusätzlich vereinfacht die Erfindung umfassende statistische Analysen, und zwar sowohl medizinischer als auch wirtschaftlicher Natur, was dazu beitragen kann, das Gesundheitswesen insgesamt ständig weiter zu verbessern.

Gemäß einem ersten Aspekt der Erfindung bezieht sich diese auf ein Verfahren zum Austausch von Gesundheitsdaten zwischen einem Computersystem und einem Nutzer, das die folgenden Schritte aufweist:
- Bereitstellen einer Nutzerdatenbank, wobei die Nutzerdatenbank Kontaktdaten des Nutzers und Gesundheitsdaten des Nutzers beinhaltet und wobei die Datenbank so strukturiert ist, dass anhand der Gesundheitsdaten eine Zuordnung des Nutzers zu einer Nutzerklasse erfolgt;
- Bereitstellen einer medizinischen Datenbank, wobei die medizinische Datenbank Einträge betreffend Nutzerklassen-spezifische Gesundheitsfragen, eine Mehrzahl von vorgegebenen Antwortmöglichkeiten zu jeder Nutzerklassen-spezifischen Gesundheitsfrage sowie Nutzerklassen-spezifische Gesundheitsempfehlungen beinhaltet;
- Bereitstellen einer Kommunikationsschnittstelle im Computersystem für Nutzer des Systems;
- Auswählen einer Nutzerklassen-spezifischen Gesundheitsfrage aus der medizinischen Datenbank für einen ausgewählten Nutzer durch das System;
- Auswählen einer Nutzerklassen-spezifischen Gesundheitsempfehlung aus der medizinischen Datenbank für den ausgewählten Nutzer durch das System;
- Versenden der ausgewählten Gesundheitsfrage vom Computersystem an den Nutzer zusammen mit der zugehörigen Mehrzahl vorgegebener Antwortmöglichkeiten;
- Empfangen einer vom Nutzer ausgewählten Antwort durch das Computersystem;
- Speichern des Gesundheitszustandes des Nutzers in der Nutzerdatenbank basierend auf den in Antwortform erhaltenen Gesundheitsdaten;
- Versenden der ausgewählten Gesundheitsempfehlung vom Computersystem an den Nutzer;
- Versenden einer Abfrage an den Nutzer, ob die an ihn gesendete Gesundheitsempfehlung von ihm umgesetzt wurde, nach Verstreichen eines vordefinierten Empfehlungszeitintervalls;
- Empfangen der Nutzerantwort zur Umsetzung der Gesundheitsempfehlung;
- Speichern des Nutzerverhaltens zur Umsetzung der Gesundheitsempfehlung in der Nutzerdatenbank.

Das erfindungsgemäße Computersystem kann aus einer oder mehreren Systemkomponenten bestehen. Bevorzugt handelt es sich bei dem Computersystem um einen Server bzw. um ein System, dessen Herzstück ein Server ist. Ein Server bietet die beste Infrastruktur, um über entsprechende Kommunikationsschnittstellen des Systems mit einem Nutzer oder anderen Kommunikationspartnern zu kommunizieren bzw. einen Datenaustausch zu ermöglichen.

Gemäß der verwendeten Definition in dieser Patentanmeldung ist ein Nutzer eine natürliche Person, die über eine Kommunikationsschnittstelle mit dem Computersystem Gesundheitsinformation bzw. Daten austauschen kann. Der Nutzer kann ein Patient sein, es ist aber nicht erforderlich, dass der Nutzer tatsächlich krank ist. Insofern wird der neutralere Begriff des Nutzers im Rahmen dieser Patentanmeldung dem Begriff des Patienten vorgezogen. Außerdem geht es im Rahmen dieser Patentanmeldung primär um eine spezielle Art von Kommunikation bzw. Kommunikationstechnologie und nicht etwa um medizinische Verfahren im engeren Sinne. Der Nutzer wiederum kann mit dem Computersystem über eine entsprechende Kommunikationsstelle kommunizieren. Dies kann beispielsweise über eine Software geschehen, die auf einem Computer, Laptop, Tablet oder auch Smartphone läuft. Bevorzugt ist es dabei so, dass der Nutzer eine tragbare Vorrichtung für Kommunikationszwecke mit dem System verwendet, da es auf diese Weise möglich ist, dass das Computersystem praktisch zu jedem Zeitpunkt an jedem Ort eine Interaktion mit dem Nutzer triggern kann. Die Tatsache, dass gemäß der vorliegenden Erfindung die Kommunikation vom Computersystem und nicht vom Nutzer ausgeht, ist sehr wichtig. Nach erstmaliger Registrierung braucht ein Nutzer von sich aus nicht mehr aktiv zu werden, sondern wird seinerseits automatisch vom System kontaktiert. Ein Nutzer kann also seine Gesundheit während des normalen Tagesablaufes nicht versehentlich oder unachtsam vergessen oder vernachlässigen, sondern wird ganz gezielt an seine Gesundheit bzw. an gesundheitsförderndes Verhalten erinnert und dabei sogar aktiv unterstützt.

Erfindungsgemäß erfolgt das Bereitstellen einer Nutzerdatenbank, wobei die Nutzerdatenbank Kontaktdaten des Nutzers und Gesundheitsdaten des Nutzers beinhaltet und wobei die Datenbank so strukturiert ist, dass anhand der Gesundheitsdaten eine Zuordnung des Nutzers zu mindestens einer Nutzerklasse erfolgt. Die Gesundheitsdaten des Nutzers können dabei bevorzugt in unterschiedlicher Ausführlichkeit in die Nutzerdatenbank eingepflegt sein. Ein Nutzer kann also in der Praxis wählen, wie vollumfänglich er das System informieren bzw. für sich nutzen möchte. Auch ein vollkommen gesunder Mensch kann als Gesundheitsdaten seine wichtigsten Kenndaten wie Alter, Gewicht, Größe, Geschlecht etc. in die Nutzerdatenbank einpflegen bzw. einpflegen lassen. Es ist aber auch möglich und für einen Nutzer von Vorteil, dass er in Zusammenarbeit mit seinem Arzt vollständige Gesundheitsdaten und insbesondere eine komplette Anamnese in die Nutzerdatenbank einpflegt bzw. einpflegen lässt. Die Architektur der Nutzerdatenbank ist dafür entsprechend ausgelegt. Anhand der Gesundheitsdaten erfolgt nun eine Zuordnung des jeweiligen Nutzers zu mindestens einer bestimmten Nutzerklasse. Diese Zuordnung kann automatisch durch das Computersystem erfolgen, sie kann aber auch händisch zum Beispiel in Zusammenarbeit mit einem Mediziner festgelegt werden. Dabei ist es möglich, dass die Nutzerklassen fortlaufend oder aber hierarchisch mit Haupt- und Unterklassen definiert sind. Eine solche Haupt- oder Oberklasse können beispielsweise Herz-/Kreislauferkrankungen bilden, deren besondere Ausprägungen wiederum anhand einer Unterklasse klassifiziert werden. Je feiner die Definition einer Nutzerklasse erfolgt, desto genauer kann das erfindungsgemäße Verfahren und System zum Wohle des Nutzers verwendet werden. Typischerweise sind zum Beispiel zwischen 1 und 20 Unterklassen zu einer Grunderkrankung vorgesehen, es können aber auch mehr Unterklassen vorgesehen sein.

Erfindungsgemäß erfolgt des Weiteren das Bereitstellen einer medizinischen Datenbank, wobei die medizinische Datenbank Einträge betreffend Nutzerklassen-spezifische Gesundheitsfragen, eine Mehrzahl von vorgegebenen Antwortmöglichkeiten zu jeder Nutzerklassen-spezifischen Gesundheitsfrage sowie Nutzerklassen-spezifische Gesundheitsempfehlungen beinhaltet. Die Einträge in der medizinischen Datenbank gliedern sich also ganz allgemein in einerseits Gesundheitsfragen, in zugehörige vorgegebene Antwortmöglichkeiten (Multiple Choice) sowie in Gesundheitsempfehlungen. Die Nutzerklassen-spezifischen Gesundheitsfragen einerseits sowie die Nutzerklassenspezifischen Gesundheitsempfehlungen andererseits sind von ihrer Natur her für eine oder mehrere Nutzerklassen geeignet und werden auch nur in Zusammenhang mit den speziell zulässigen Nutzerklassen überhaupt an einen Nutzer versendet. Es ist eine große Stärke des erfindungsgemäßen Verfahrens, dass neben den gestellten Gesundheitsfragen bereits vorgegebene Antwortmöglichkeiten in der medizinischen Datenbank enthalten sind. Die Frage und die zugehörigen Antwortmöglichkeiten bilden dabei einen zusammengehörenden Frage-Antwort-Datensatz. Dies macht es für einen Nutzer einfacher, die Frage zu beantworten. Die Antwort ist klar und unmissverständlich. Das wiederum erleichtert eine entsprechende individuelle oder auch statistische Auswertung der erhaltenen Antworten. Antworten verschiedener Nutzer werden im Rahmen einer statistischen Analyse vergleichbarer bzw. statistisch aussagekräftiger. Die Zahl vorgegebener Antwortmöglichkeiten kann je nach Frage variieren. Es ist nicht erforderlich, dass stets dieselbe Anzahl von vorgegebenen Antwortmöglichkeiten für Frage-Antwort-Datensätze verwendet wird. Natürlich kann dies aber so sein. Dabei ist es bevorzugt so, dass nicht lediglich Ja/Nein-Antworten möglich sind, sondern dass die Antwortmöglichkeiten differenzierter ausgestaltet sind. Typischerweise können 3 bis 5 verschiedene Antwortmöglichkeiten oder aber auch mehr Antwortmöglichkeiten zu einer Gesundheitsfrage in der medizinischen Datenbank als Datensatz abgespeichert sein.

In der Datenbank können auch solche Gesundheitsfragen, dazugehörige Antwortoptionen und/oder solche Gesundheitsempfehlungen abgelegt sein, die auf alle Nutzer zutreffen bzw. allen Nutzern gestellt werden können. Diese Fragen, Antworten und Empfehlungen sind dann also allen Nutzerklassen zugewiesen und werden als allgemeine Gesundheitsfragen, - Antworten bzw. Empfehlungen bezeichnet. Im Rahmen der Erfindung ist aber mindestens ein Datensatz zu Fragen mit Antwortmöglichkeiten und mindestens ein Datensatz Empfehlungen in der medizinischen Datenbank enthalten, die echt Nutzerklassen-spezifisch im engeren Sinne und damit nicht allgemein für alle Nutzer verwendbar sind. Die Nutzerklassen-Spezifizität ist eine echte Stärke der Erfindung. Typischerweise sind zwischen 10% und 90% aller Frage-Antwort-Datensätze und/oder zwischen 10% und 90% aller Empfehlungsdatensätze Nutzerklassen-spezifisch im engeren Sinne. Bevorzugt sind mindestens 50%, höchst bevorzugt mindestens 90% aller Frage-Antwort-Datensätze und/ oder mindestens 50%, höchst bevorzugt mindestens 90% aller Empfehlungsdatensätze Nutzerklassen-spezifisch im engeren Sinne. Im Rahmen dieser Patentanmeldung sind die Begriffe Gesundheit bzw. Gesundheitsfragen und Gesundheitsempfehlungen bevorzugt weit auszulegen. Das heißt, dass alle Fragen und Empfehlungen, die einen sinnvollen Bezug zur Gesundheit haben, auch als Gesundheitsfrage bzw. Gesundheitsempfehlung gelten. Der Begriff Gesundheit umfasst also nicht nur den engeren Bereich der Medizin, sondern auch die Bereiche Sport, Ernährung, Lebensstil etc., mithin Bereiche, die einen maßgeblichen Einfluss auf die Gesundheit von Personen haben können.

Gemäß dem erfindungsgemäßen Verfahren erfolgt ein Bereitstellen einer Kommunikationsstelle im Computersystem für Nutzer des Systems. Über eine solche Kommunikationsschnittstelle kann das System bevorzugt mit mehreren Nutzern gleichzeitig kommunizieren. Es ist insbesondere auch möglich, dass ein Nutzer über die Kommunikationsstelle dauerhaft mit dem Computersystem verbunden ist, der Nutzer also im System eingeloggt bleibt. Dies erleichtert die vom Computersystem getriggerte Kommunikation mit dem Nutzer erheblich. Auch ist es so, dass zum Beispiel in sozialen Netzwerken viele Verwender dieser Netzwerke ohnehin ständig in diesen Netzwerken eingeloggt sind, zum Beispiel über ihre Mobiltelefone bzw. Smartphones.

Erfindungsgemäß erfolgt dann das Auswählen einer Nutzerklassen-spezifischen Gesundheitsfrage aus der medizinischen Datenbank für einen ausgewählten Nutzer durch das System. Es wird also ganz individuell für einen bestimmten Nutzer eine auf ihn zugeschnittene Gesundheitsfrage automatisch durch das System ausgewählt. Anknüpfungspunkt für diese Auswahl sind die in der Nutzerdatenbank gespeicherten Gesundheitsdaten des Nutzers bzw. daraus abgeleitet die Zuordnung des Nutzers zu einer bestimmten Nutzerklasse. Es ist dabei beispielsweise möglich, dass das System für einen Nutzer einer bestimmten Nutzerklasse jedes Mal bei einer täglichen Erstkommunikation mit dem Nutzer dieselbe Gesundheitsfrage aus der medizinischen Datenbank auswählt. Es ist aber auch möglich, dass die Auswahl bestimmter Gesundheitsfragen, die für eine bestimmte Nutzerklasse in Frage kommen, nach einem bestimmten Algorithmus oder nach einem Zufallsverfahren erfolgt. Welche Gesundheitsfrage auch immer für einen Nutzer einer bestimmten Nutzerklasse gewählt wird, diese Gesundheitsfrage passt zum jeweiligen Nutzer und unterstützt diesen beim Erhalt oder der Wiederherstellung seiner Gesundheit. Im weiteren Verlauf ist es auch möglich, dass vom Nutzer gegebenen Antworten zu den Gesundheitsfragen die Auswahl einer weiteren Gesundheitsfrage durch das System beeinflussen und die Auswahl also auf einer Nutzerantwort basiert. Entsprechende Algorithmen können in dem erfindungsgemäßen Verfahren implementiert werden.

Ähnlich verhält es sich beim Verfahrensschritt Auswählen einer Nutzerklassen-spezifischen Gesundheitsempfehlung aus der medizinischen Datenbank für den ausgewählten Nutzer durch das System. Auch hier erhält der Nutzer in jedem Fall eine an seine Nutzerklasse angepasste Auswahl, die vom System automatisch getroffen wird. Auch hierbei können bereits vorher von einem Nutzer gegebene Gesundheitsantworten mit berücksichtigt werden. Bei der Gesundheitsempfehlung handelt es sich bevorzugt um eine konkrete Handlungsempfehlung, die ein Nutzer kurzfristig, zum Beispiel am selben Tag oder in den nächsten Stunden, ausführen kann.

In einem weiteren Verfahrensschritt erfolgt dann das Versenden der ausgewählten Gesundheitsfrage vom Computersystem an den Nutzer zusammen mit der zugehörigen Mehrzahl vorgegebener Antwortmöglichkeiten. In der Praxis ist es beispielsweise so, dass in einem optisch ansprechenden Format die entsprechende Gesundheitsfrage auf einem Datenendgerät, das vom Nutzer verwendet wird, zusammen mit der Mehrzahl der Antwortmöglichkeiten angezeigt wird. Das Versenden der ausgewählten Gesundheitsfrage erfolgt wiederum durch das Computersystem automatisch. Das Versenden kann zu einem durch das System ausgewählten Zeitpunkt erfolgen, es ist aber auch möglich, dass ein solcher Versand dann stattfindet, wenn sich ein Nutzer erneut mit dem Computersystem verbindet bzw. sich in dieses System einloggt. Zielführender ist aber auch hier das ständige eingeloggt sein des Nutzers im System, um den Versand der Gesundheitsfrage vom Computersystem an den Nutzer optimal zu triggern. Gemäß einer bevorzugten Ausführungsform der Erfindung ist es so, dass das System den Zeitpunkt zum Versenden der ausgewählten Gesundheitsfrage basierend auf Angaben des Nutzers zu seinem Tagesablauf und seinen Gewohnheiten festlegt. Diese werden zum Beispiel bei der Neuregistrierung eines Nutzers abgefragt. Das System analysiert diese Angaben und ermittelt dann einen optimalen Zeitpunkt für das Versenden. Dabei kann zum Beispiel das Versenden jeden Tag zur selben Zeit erfolgen, es kann aber auch sein, dass das Versenden an verschiedenen Wochentagen zu unterschiedlichen Zeiten erfolgt. Auf diese Weise kann das erfindungsgemäße Verfahren einen Nutzer desselben auch zeitlich optimal unterstützen.

In einem nächsten Schritt erfolgt das Empfangen einer vom Nutzer ausgewählten Antwort durch das Computersystem. Der Nutzer hat also aus den vorgeschlagenen Antwortmöglichkeiten eine für sich passende ausgewählt. Bevorzugt ist es dabei so, dass der Nutzer die von ihm ausgewählte Antwort an das Computersystem zeitnah nach Erhalt der Gesundheitsfrage versendet. Dies ist ein Indiz für eine engagierte Nutzung des Systems durch den Nutzer. Es ist auch möglich, ein bestimmtes Zeitintervall zu definieren, innerhalb dem die Antwort vom Nutzer beim Computersystem eingegangen sein muss. Andernfalls gilt die Antwort als nicht gegeben. Dies ist insbesondere dann wichtig, wenn das Nutzungsverhalten des Nutzers analysiert wird bzw. wenn bei einem Scoring bzw. Belohnungssystem der Nutzer aus der Nutzung des Systems für sich weitere Vorteile ziehen möchte.

Anschließend erfolgt das Speichern des Gesundheitszustandes in der Nutzerdatenbank basierend auf den in Antwortform erhaltenen Gesundheitsdaten. Dabei ist es möglich, dass die Daten selbst in der Nutzerdatenbank abgespeichert werden, es sind dann also exakte Angaben über den Gesundheitszustand des Nutzers, zum Beispiel seinen Blutdruck, seinen Insulinspiegel etc. in der Nutzerdatenbank enthalten. Es ist aber auch möglich, dass vor der eigentlichen Speicherung eine Analyse oder Auswertung der Gesundheitsdaten erfolgt und nur pauschaler Angaben wie "Werte okay" oder im Gegenteil "Werte nicht okay" in der Nutzerdatenbank abgespeichert werden.

In einem weiteren Verfahrensschritt erfolgt das Versenden der ausgewählten Gesundheitsempfehlung vom Computersystem an den Nutzer. Das Versenden der ausgewählten Gesundheitsempfehlung kann dabei gleich nach Erhalt einer Nutzerantwort auf eine Gesundheitsfrage hin erfolgen. Es ist aber auch möglich, dass sowohl die Gesundheitsfrage mit den vorgegebenen Antwortoptionen als auch die Gesundheitsempfehlung gleichzeitig an den Nutzer versendet werden. Natürlich ist es auch möglich, erst die Gesundheitsempfehlung und erst zu einem späteren Zeitpunkt dann die Gesundheitsfrage an den Nutzer zu versenden. Insofern ist die Reihenfolge der einzelnen Verfahrensschritte des erfindungsgemäßen Verfahrens nicht strikt vorgegeben bzw. nicht strikt an die Reihenfolge der Abfassung der einzelnen Verfahrensschritte im Patentanspruch gekoppelt.

In einem weiteren Verfahrensschritt erfolgt das Versenden einer Abfrage an den Nutzer, ob die an ihn gesendete Gesundheitsempfehlung von ihm umgesetzt wurde, und zwar nach Verstreichen eines vordefinierten Empfehlungszeitintervalls. Die Definition eines solchen Empfehlungszeitintervalls ist sehr wichtig, denn natürlich braucht ein Nutzer für die Umsetzung einer Gesundheitsempfehlung etwas Zeit. Es geht bei der Gesundheitsempfehlung nicht primär darum, abzufragen, was der Nutzer ohnehin schon für seine Gesundheit getan hat, sondern es geht darum, ihn in seinem Tagesablauf bei Maßnahmen zum Erhalt seiner Gesundheit aktiv zu unterstützen. Bei der Abfrage an den Nutzer handelt es sich im einfachsten Fall um eine Frage, die eine einfache Ja oder Nein-Antwort erfordert. Es ist auch möglich, dass im Falle einer bejahenden Antwort vom System noch eine eingehendere Nachfrage an den Nutzer versendet wird, die darauf abzielt, den Grad der Umsetzung der Gesundheitsempfehlung genauer auszuloten. Beispielsweise könnte eine Gesundheitsempfehlung lauten: Gehen Sie heute ein paar Minuten spazieren! Auf die Abfrage: Sind Sie heute spazieren gegangen? könnte dann als Nachfrage vom System an den Nutzer versendet werden, wie viele Minuten oder welche Strecke er denn spazieren gegangen ist. Dies könnte wiederum in Form einer Frage mit mehreren vorgegebenen Antwortmöglichkeiten geschehen.

Nach Empfangen der Nutzerantwort zur Umsetzung der Gesundheitsempfehlung erfolgt ein Speichern des Nutzerverhaltens zur Umsetzung der Gesundheitsempfehlung in der Nutzerdatenbank. Es kann beispielsweise der Wortlaut der Gesundheitsempfehlung selbst und die Umsetzung durch den Nutzer in Form eines Positiv- oder Negativeintrages in der Nutzerdatenbank abgespeichert werden. Es ist auch möglich, lediglich die Anzahl der umgesetzten Gesundheitsempfehlungen in der Nutzerdatenbank abzuspeichern. Es ist möglich, die Anzahl der umgesetzten Gesundheitsempfehlungen zu einer Anzahl von nicht umgesetzten Gesundheitsempfehlungen in Relation zu setzen. Bevorzugt ist es so, dass das Nutzerverhalten zur Umsetzung einer Gesundheitsempfehlung sehr ausführlich in der Nutzerdatenbank gespeichert wird.

Gemäß einer bevorzugten Ausführungsform der Erfindung erfolgt das Bewerten des Nutzers hinsichtlich seines Nutzungsverhaltens des Systems und insbesondere hinsichtlich seiner Ausführung von Gesundheits- bzw. Handlungsempfehlungen. Eine solche Bewertung kann nach jeder Interaktion des Systems mit einem Nutzer oder in regelmäßigen Zeitabständen-z. B, einmal täglich oder wöchentlich - oder auch erst auf Anfrage eines Nutzers an das System oder auf Anfrage eines Dritten erfolgen. Es geht bei diesem Bewertungsschritt nicht primär darum festzustellen, ob der Gesundheitszustand des Nutzers gut oder schlecht ist. Es wird vielmehr objektiv bewertet, ob der Nutzer eines Systems aktiv an dem Verfahren teilnimmt, das heißt Gesundheitsfragen beantwortet und Gesundheitsempfehlungen befolgt. Handelt er aktiv und positiv für seine Gesundheit, so erfolgt hierfür eine positive Bewertung. Das Bewerten selbst kann dabei im Rahmen eines Punktesystems (Scoring) erfolgen. Es ist beispielsweise möglich, für jede befolgte Gesundheitsempfehlung einen bestimmten Punktwert zu vergeben. Der dazugehörige Punktwert kann beispielsweise in der medizinischen Datenbank hinterlegt sein. Es ist möglich, für verschiedene Gesundheitsempfehlungen bzw. für deren Umsetzung verschieden viele Punkte zu vergeben. Es gibt also sozusagen unterschiedliche Schwierigkeitsstufen bei der Befolgung von Gesundheitsempfehlungen. Ein aktives und gesundheitsbewusstes Verhalten wird so im Rahmen des erfindungsgemäßen Verfahrens objektiv positiv bewertet.

Dabei ist es möglich, dass in einem weiteren Verfahrensschritt einem Nutzer seine aktuelle Bewertung mitgeteilt wird. Es kann beispielsweise eine entsprechende Nachricht an ihn versandt werden. Es ist auch möglich, dass kein automatischer Versand erfolgt, der Nutzer aber jederzeit über eine entsprechende Abfrage seiner in der Nutzerdatenbank gespeicherten Daten prüfen kann, welchen Score bzw. Punktwert er aktuell erreicht. Eine positive Bewertung spornt einen Nutzer zur weiteren Verwendung des Systems an, insbesondere dann, wenn für ihn damit andere handfeste wirtschaftliche Vorteile verknüpft werden können. Es ist beispielsweise denkbar, dass über entsprechende Verträge ein Krankenversicherer günstigere Beiträge für einen Nutzer anbietet, der aktiv Vorsorge für seine Gesundheit trifft bzw. sich grundsätzlich gesundheitsbewusst ernährt und verhält.

Gemäß einer bevorzugten Ausführungsform der Erfindung wird eine Nutzerklasse anhand wenigstens einem der folgenden Kriterien definiert: Alter des Nutzers, Geschlecht des Nutzers, Erkrankung, Schweregrad einer Erkrankung, Komplikation bei einer Erkrankung, Medikation. Auch der Lebensstil kann zur Definition einer Nutzerklasse dienen. Die anhand eines solchen Kriteriums definierte Nutzerklasse kann eine eigenständige Nutzerklasse, eine Haupt-Nutzerklasse oder eine Unter-Nutzerklasse definieren. Es ist beispielsweise möglich, zum Beispiel über eine bestimmte Erkrankung eine Hauptnutzerklasse zu definieren, weitere Unterklassen können dann den Schweregrad der entsprechenden Erkrankung, eine Komplikation bei dieser Erkrankung und die entsprechende Medikation definieren. In diesem Beispiel gibt es dann also eine Hauptklasse sowie 3 Unterklassen. Natürlich ist eine feinere oder eine gänzlich andere Untergliederung der Nutzerklassen möglich. Gemäß einer bevorzugten Ausführungsform wird eine Nutzerklasse durch eine Kombination aus einer Hauptklasse mit mindestens einer Unterklasse definiert. Bevorzugt folgt diese Nutzerklassendefinition einer hierarchischen bzw. Baumstruktur. Insgesamt ist die Nutzerklassendefinition davon abhängig zu machen, wie komplex und detailliert das erfindungsgemäße Verfahren bzw. Computersystem arbeiten soll.

Gemäß einer bevorzugten Ausführungsform der Erfindung weist das Verfahren des Weiteren den folgenden Schritt auf: Regelmäßiges Überprüfen der Zuordnung eines Nutzers zu einer Nutzerklasse und gegebenenfalls entsprechendes Aktualisieren seiner Nutzerklasse. Es kann dazu zum Beispiel so sein, dass nach einer Erstregistrierung eine Erstklassifikation des Nutzers erfolgt. Diese Erstklassifikation kann dann später überprüft und - falls erforderlich-verfeinert werden. Das heißt, es wird zum Beispiel mit einer Hauptklasse gestartet, die dann zum Beispiel später im Rahmen der Nutzung des Verfahrens um eine oder mehrere Unterklassen ergänzt wird. Auch können falls erforderlich natürlich Klassen gänzlich getauscht bzw. aktualisiert werden. Das erfindungsgemäße Verfahren ist also auch in Hinblick auf eine Nutzerklassenzuordnung eines Nutzers Ausbau und lernfähig.

Gemäß einer bevorzugten Ausführungsform der Erfindung ist in der medizinischen Datenbank mindestens einer Gesundheitsfrage, insbesondere allen Gesundheitsfragen, ein Wiederholungszeitintervall als Parameter zugeordnet, der angibt, in welchen zeitlichen Abstände dieselbe Gesundheitsfrage an denselben Nutzer erneut versendet wird. Es gibt zum Beispiel Gesundheitsfragen, die täglich, alle 2 Tage oder wöchentlich etc. wiederholt demselben Nutzer gestellt werden. Es handelt sich dabei also um eine in einem speziellen Turnus wiederkehrende Gesundheitsfrage. Dabei können verschiedene Gesundheitsfragen ein unterschiedliches Wiederholungzeitintervall aufweisen. Das Wiederholungszeitintervall kann also ganz individuell für die jeweilige Gesundheitsfrage sinnvoll definiert werden.

Gemäß einer bevorzugten Ausführungsform der Erfindung umfassen die in der medizinischen Datenbank hinterlegten Parameter sowohl objektive als auch subjektive Gesundheitsparameter eines Nutzers. Die Gesundheitsparameter werden dabei insbesondere durch die möglichen Antworten auf eine bestimmte Gesundheitsfrage realisiert. Diese Antwortmöglichkeiten gehören zu einem bestimmten Typ und können in objektive und subjektive Gesundheitsparameter unterteilt werden. Alle Antwortoptionen zu einer bestimmten Gesundheitsfrage gehören bevorzugt demselben Parametertyp an. Unter einem objektiven Gesundheitsparameter wird ein messbarer Gesundheitsparameter verstanden. Zu diesen zählt beispielsweise der Blutdruck, der Puls, das Körpergewicht, der Körperfettanteil, der Blutzucker bzw. Insulinspiegel etc. Aber auch subjektive Gesundheitsparameter eines Nutzers zählen bevorzugt zu den in der medizinischen Datenbank hinterlegten Gesundheitsdaten. Auf die Gesundheitsfrage: "Wie haben Sie letzte Nacht geschlafen?" kann ein Nutzer im Prinzip nur eine subjektive Antwort geben, die sein Empfinden der Situation bestmöglich wiedergibt. Er kann beispielsweise antworten, er habe sehr schlecht, schlecht, gut oder sehr gut geschlafen. In objektive Hinsicht wäre nur eine bestimmte Schlafdauer festzustellen gewesen. Dieser objektive Parameter gibt aber das subjektive Empfinden eines Nutzers zum Thema Schlaf nur unzureichend wieder. Aus diesem Grunde werden zum Erhalt eines umfassenden Gesundheitsbildes bevorzugt auch die subjektiven Parameter eines Nutzers als Gesundheitsparameter miterfasst, es ist dann also mindestens ein entsprechender Datensatz dieses subjektiven Typs vorhanden. Außerdem hat die Implementierung von subjektiven Gesundheitsparametern in das erfindungsgemäße Verfahren den positiven Effekt, dass ein Nutzer die Gesundheitsfragen als mehr auf seine Person abgestimmt und als persönlicher empfindet. Es geht eben nicht nur um abstrakte Werte, sondern um sein ganz persönliches gesundheitliches Befinden. Wird dieser Aspekt eines Nutzers angesprochen, so ist die Mitarbeit eines Nutzers bei dem erfindungsgemäßen Verfahren besser und das Verfahren kann die Gesundheit eines Nutzers besser und positiver unterstützen.

Gemäß einer bevorzugten Ausführungsform des Verfahrens ist es so, dass das Auswählen einer Gesundheitsfrage und/oder das Auswählen einer Gesundheitsempfehlung durch das System basierend auf einem Feed-Back-Loop erfolgt. Dabei wird dann also beim Auswählen auf Daten zurückgegriffen, die in Form von Nutzerantworten zu den Gesundheitsfragen und/ oder in Form von Nutzerangaben zur Umsetzung von Gesundheitsempfehlungen vom System erhalten wurden. Dies ermöglicht eine noch bessere Abstimmung des Verfahrens auf den jeweiligen Nutzer und seine Bedürfnisse.

Gemäß einer weiteren Ausführungsform der Erfindung weist das erfindungsgemäße Verfahren des Weiteren den folgenden Verfahrensschritt auf:
- Bereitstellen einer weiteren Kommunikationsschnittstelle für einen weiteren Akteur des Gesundheitswesens, insbesondere für einen Arzt, ein Krankenhaus, eine Apotheke, einen Krankenversicherer.
   Die vorgenannte Liste ist dabei nicht abschließend. Sie verdeutlicht aber, dass das erfindungsgemäße Verfahren sehr umfassend im besten Fall alle Akteure des Gesundheitswesens in das Verfahren mit integrieren kann. Dabei ist es natürlich nicht so, dass jeder Akteur des Gesundheitswesens einen vollständigen Zugriff auf die Nutzerdatenbank oder die medizinische Datenbank hat. Stattdessen ist es so, dass je nach Art bzw. Klasse des Akteurs ein entsprechender Zugriff auf bestimmte Daten erfolgen kann. Es ist also möglich, dass über diese weitere Kommunikationsschnittstelle ein Akteur aktiv, das heißt von sich auch, gemäß seiner Zugriffsrechte den Eintrag eines Nutzers in einer Datenbank des Computersystems liest. Zusätzlich oder alternativ ist es möglich, dass sogar Schreibrechte für das System vergeben werden. Beispielsweise kann es so sein, dass ein Arzt nach dem Verschreiben einer bestimmten Medikation in Einverständnis mit seinem Patienten bzw. Nutzer des erfindungsgemäßen Verfahrens die verschriebene Medikation in die Nutzerdatenbank unter den Gesundheitsdaten des Nutzers einträgt. Das erfindungsgemäße Verfahren ist also in vielerlei Hinsicht erweiterbar. Zusätzlich oder alternativ ist es möglich, dass über die weitere Kommunikationsschnittstelle lediglich unidirektional eine Kommunikation des Computersystems nach außen hin zu dem weiteren Akteur erfolgt. Unter diesem Aspekt handelt es sich also um die gezielte (und mit dem Nutzer abgestimmte und von diesem autorisierte) Information/Datenweitergabe an einen weiteren Teilnehmer bzw. Akteur des Systems.

Gemäß einer weiteren bevorzugten Ausführungsform der Erfindung kann ein automatisches Austauschen von Gesundheitsdaten betreffend einen Nutzer zwischen dem Computersystem und dem weiteren Akteur des Gesundheitswesens erfolgen. Dies geschieht in der Praxis natürlich unter Berücksichtigung von Datenschutzbestimmungen und Datenzugriffsrechten des weiteren Akteurs im Computersystem. Bevorzugt ist es dabei so, dass das Austauschen von Gesundheitsdaten betreffend einen Nutzer zwischen dem Computersystem und mehreren Akteuren des Gesundheitswesens gleichzeitig und insbesondere in Echtzeit erfolgt. Dabei ist auch der Fall mitumfasst, dass in der Nutzerdatenbank gespeicherte Gesundheitsdaten des Nutzers zeitglich dem Nutzer selbst und einem Akteur des Gesundheitswesen, zum Beispiel dem Arzt des Nutzers, mitgeteilt werden. Dadurch ist es möglich, verschiedene Akteure des Gesundheitswesens gleichzeitig auf dem neuesten Stand hinsichtlich der Gesundheit eines Nutzers zu halten. Das gleichzeitige Austauschen von Gesundheitsdaten kann dabei Gesundheitsdaten im engeren Sinne, aber auch die Bewertung des Nutzungsverhaltens des Systems durch den Nutzer beinhalten. Es ist beispielsweise möglich, dass ein Arzt, ein Krankenhaus, eine Apotheke oder ein Krankenversicherer zeitgleich vom Computersystem über den aktuellen Punktwert des Nutzers zum Nutzungsverhalten des Nutzers informiert werden, damit der Nutzer bei einem nachfolgenden Besuch in der Apotheke, bei Arzt, beim Krankenhaus oder beim Krankenversicherer eine diesem Punktwert entsprechende Prämie einlösen kann. In der Praxis ist z.B. in der Nutzerdatenbank ein entsprechender Eintrag hinterlegt, welcher Akteur ggf. auf welchem Level für den betreffenden Nutzer noch mit in das Gesamtverfahren bzw. System integriert ist.

Gemäß einer weiteren bevorzugten Ausführungsform der Erfindung weist das erfindungsgemäße Verfahren zusätzlich den folgenden Schritt auf:
- Analysieren der Wirksamkeit und/oder der Wirtschaftlichkeit von gesundheitsfördernden Maßnahmen.
   Dabei können durch an sich bekannte statistische Methoden Daten aus der Nutzerdatenbank und/oder aus der medizinischen Datenbank untersucht werden. Es lässt sich beispielsweise feststellen, ob eine bestimmte Medikation gegen eine bestimmte Krankheit überdurchschnittlich erfolgversprechend oder aber mit überdurchschnittlich viel Nebenwirkungen verknüpft ist. Auch der ansonsten nur schwer feststellbare Einfluss der gesunden Lebensweise auf das Auftreten oder den Schweregrad von Krankheiten lässt sich basierend auf dem erfindungsgemäßen Verfahren besser untersuchen. Diese so genannten weichen Parameter sind in herkömmlichen Verfahren nämlich praktisch nicht greifbar und schon gar nicht statistisch aussagekräftig bzw. statistisch signifikant auswertbar.

Das oben beschriebene erfindungsgemäße Verfahren und seine bevorzugten Ausführungsformen können ganz oder teilweise miteinander kombiniert werden, sofern sich dadurch nicht technische Widersprüche ergeben. Insbesondere ist es möglich, dass das erfindungsgemäße Verfahren ganz oder teilweise mehrfach ausgeführt wird. Der zuständige Fachmann wird erkennen, welche Kombinationen technisch machbar und sinnvoll sind. Die Reihenfolge der einzelnen Verfahrensschritte kann variieren, sofern einzelne Verfahrensschritte nicht voneinander abhängig sind bzw. aufeinander aufbauen.

Gemäß einem weiteren Aspekt der Erfindung bezieht sich diese auf ein Computerprogrammprodukt mit einem Programmcode zum Ausführen des Verfahrens wie oben allgemein oder hinsichtlich spezieller Ausführungsformen beschrieben. Das Computerprogrammprodukt kann dabei in körperhafter oder nicht-körperhafter Form vorliegen. Der Programmcode muss nicht in einer bestimmten Programmiersprache verfasst sein. Es sollte lediglich eine der Problemstellung angemessene Programmiersprache verwendet werden. Das Erfindungsgemäße am Computerprogrammprodukt liegt nicht in dem Programmcode selbst, sondern in dem dem Programmcode zugrundeliegenden technischen Verfahren wie oben beschrieben. Das Computerprogrammprodukt kann insbesondere einteilig oder mehrteilig, zum Beispiel mit einem modular untergliederten Programmcode, abgefasst sein.

Die Programmiersprachen zur Programmierung des Verfahrens können zum Beispiel eine Kombination aus Standardprogrammiersprachen und Standards wie z. B. HTML5, Java, JavaScript, sein. Das ermöglicht das Ausführen des Verfahrens unabhängig von dem jeweiligen Betriebssystem (Windows, Linux und seine Arten, Android, iOS, usw.) Die Liste der Programmiersprachen ist nicht festgelegt und wird sich auch mit der Weiterentwicklung der Computertechnik verändern.

Gemäß einem weiteren Aspekt der Erfindung bezieht sich diese auf ein System zum Austausch von Gesundheitsdaten zwischen einem Server und einem Nutzer, das Folgendes aufweist:
- Eine Nutzerdatenbank mit Nutzerinformation betreffend Kontaktdaten des Nutzers und betreffend Gesundheitsdaten des Nutzers, wobei die Datenbank so strukturiert ist, dass anhand der Gesundheitsdaten eine Zuordnung des Nutzers zu mindestens einer Nutzerklasse erfolgt;
- eine medizinische Datenbank mit Nutzerklassen-spezifischen Gesundheitsfragen und jeweils mehreren möglichen zugehörigen Antworten sowie mit Nutzerklassen-spezifischen Gesundheitsempfehlungen;
- einen Programmcode zum Kommunikationsmanagement zwischen dem Server und dem Nutzer;
- einen Programmcode zur automatischen Gesundheitsunterstützung, der programmiert ist, um automatisch eine an den Nutzer zu versendende Nutzerklassen-spezifische Gesundheitsfrage mit zugehörigen möglichen Antwortoptionen auszuwählen, und der weiter eingerichtet, eine an den Nutzer zu versendende Nutzerklassen-spezifische Handlungsempfehlung für den Nutzer auszuwählen;
- einen Programmcode zur automatischen Gesundheitszustandserfassung des Nutzers basierend auf vom Nutzer an den Server in Antwortform übermittelten Gesundheitsdaten; und
- einen Programmcode zur automatischen Ausführungskontrolle von erhaltenen Gesundheitsempfehlungen basierend auf vom Nutzer an den Server übermittelten Angaben.

Das erfindungsgemäße System zum Austausch von Gesundheitsdaten ist insbesondere dafür geeignet, das erfindungsgemäße Verfahren zum Austausch von Gesundheitsdaten zwischen einem Computersystem und einem Nutzer wie oben beschrieben auszuführen. Die oben erwähnten Programmcodes sind genauso eingerichtet bzw. programmiert, dass die entsprechenden Verfahrensschritte vom Computersystem bzw. dem Server automatisch abgearbeitet werden können. Bevorzugt sind einige oder alle Datenbanken und einige oder alle Programmcodes auf dem Server abgelegt, d.h. also der Server weist die Datenbanken und Programmcodes auf. Im Übrigen gilt für das erfindungsgemäße System das bereits oben zum erfindungsgemäßen Verfahren Ausgesagte. Erläuterungen hinsichtlich der Terminologie in Zusammenhang mit dem Verfahren gelten genauso in Zusammenhang mit dem erfindungsgemäßen System.

Ergänzend sei auf Folgendes hingewiesen:
Der Programmcode zum Kommunikationsmanagement zwischen dem Server und dem Nutzer erlaubt das Bereitstellen einer Kommunikationsschnittstelle im Computersystem für Nutzer des Systems sowie gegebenenfalls auch für andere Akteure des Gesundheitswesens. Außerdem kann bevorzugt über diesen Programmcode das Versenden und Empfangen von Daten abgewickelt werden.

Der Programmcode zur automatischen Gesundheitsunterstützung erlaubt das Auswählen von Gesundheitsfragen samt zugehörigen Antwortoptionen und Gesundheitsempfehlungen aus der medizinischen Datenbank.

Der Programmcode zur automatischen Gesundheitszustandserfassung erlaubt insbesondere das Speichern von in Antwortform vom Nutzer erhaltenen Gesundheitsdaten. Es ist bevorzugt auch möglich, auf anderem Wege erhaltene neue Gesundheitsdaten in der Nutzerbank abzuspeichern.

Der Programmcode zur automatischen Ausführungskontrolle von erhaltenen Gesundheitsempfehlungen erlaubt das Speichern des Nutzerverhaltens zur Umsetzung der Gesundheitsempfehlungen in der Nutzerdatenbank.

Gemäß einer bevorzugten Ausführungsform der Erfindung weist der Server einen Programmcode zur automatischen Bewertung des Nutzers hinsichtlich seines Nutzungsverhaltens des Systems und insbesondere für seine Ausführung von Gesundheitsempfehlungen auf. Mithilfe dieses Programmcodes kann also z.B. eine Punktbewertung bzw. ein Scoring für den jeweiligen Nutzer erstellt und/oder verwaltet werden.

Gemäß einer bevorzugten Ausführungsform des Systems ist eine Nutzerklasse anhand wenigstens einem der folgenden Kriterien definiert: Alter des Nutzer, Geschlecht des Nutzers, Erkrankung, Schweregrad einer Erkrankung, Komplikation bei einer Erkrankung, Medikation. Auch der Lebensstil von Nutzern kann zur Nutzerklassendefinition herangezogen werden.

Gemäß einer bevorzugten Ausführungsform des Systems ist eine Nutzerklasse durch eine Kombination aus einer Hauptklasse mit mindestens einer Nebenklasse definiert.

Gemäß einer bevorzugten Ausführungsform des Systems ist ein Programmcode zum regelmäßigen Überprüfen der Zuordnung eines Nutzers zu einer Nutzerklasse und zum gegebenenfalls entsprechenden Aktualisieren seiner Nutzerklasse vorgesehen.

Gemäß einer weiteren bevorzugten Ausführungsform des Systems ist in der medizinischen Datenbank mindestens einer Gesundheitsfrage, insbesondere allen Gesundheitsfragen, ein Wiederholungszeitintervall als Parameter zugeordnet, der angibt, in welchen zeitlichen Abständen dieselbe Gesundheitsfrage an denselben Nutzer erneut zu versenden ist.

Gemäß einer weiteren bevorzugten Ausführungsform des Systems umfassen die vom Server in Antwortform erhaltenen Gesundheitsdaten sowohl objektive als auch subjektive Gesundheitsparameter eines Nutzers.

Gemäß einer weiteren bevorzugten Ausführungsform des Systems wird mindestens ein Gesundheitsparameter aus der folgenden Liste verwendet: Blutdruck, Puls, Körpergewicht, Körperfettanteil, Blutzucker.

Gemäß einer weiteren bevorzugten Ausführungsform des Systems weist das System eine Kommunikationsschnittstelle für mindestens einen weiteren Akteur des Gesundheitswesens auf, insbesondere eine Kommunikationsschnittstelle für einen Arzt, ein Krankenhaus, eine Apotheke, einen Krankenversicherer.

Gemäß einer bevorzugten Ausführungsform des Systems weist der Server des Weiteren einen Programmcode zur automatischen Kommunikation zwischen dem Server und dem weiteren Akteur des Gesundheitswesen auf, wobei im Rahmen dieser Kommunikation Gesundheitsdaten betreffend einen Nutzer zwischen dem Server und dem weiteren Akteur des Gesundheitswesens ausgetauscht werden.

Gemäß einer weiteren bevorzugten Ausführungsform des Systems erfolgt ein Austausch von Gesundheitsinformation betreffend einen Nutzer des Systems zwischen dem Server und mehrere Akteuren des Gesundheitssystems gleichzeitig und insbesondere in Echtzeit.

Gemäß einer weiteren bevorzugten Ausführungsform des erfindungsgemäßen Systems weist der Server des Weiteren einen Statistikprogrammcode auf, der programmiert ist, um die Wirksamkeit und/oder Wirtschaftlichkeit von gesundheitsfördernden Maßnahmen zu analysieren.

Gemäß einer weiteren bevorzugten Ausführungsform weist das Systems ein mobiles Nutzer-Endgerät, insbesondere ein Smartphone oder Tablet, auf, über das der Nutzer mit dem System kommuniziert. Dieses mobile Nutzer-Endgerät hat den Vorteil, dass eine Kommunikation mit dem Nutzer praktisch an allen Orten und zu jeder Zeit möglich ist, ganz so wie es der bestmöglichen Förderung des Gesundheitszustandes eines Nutzers entspricht.

Auch für das erfindungsgemäße System gilt, dass eine oder mehrere Ausführungsvarianten des Systems ganz oder teilweise miteinander kombiniert werden können, sofern sich dadurch nicht technische Widersprüche ergeben.

Die Erfindung wird noch besser verstanden werden unter Bezugnahme auf die beigefügten Figuren. Dabei zeigen:
- Figur 1:: zeigt ein Flussdiagramm des erfindungsgemäßen Verfahrens zum Austausch von Gesundheitsdaten;
- Figur 2:: zeigt exemplarisch und in schematischer Darstellung den Aufbau einer Nutzerdatenbank gemäß der Erfindung;
- Figur 3:: zeigt schematisch einen exemplarischen Aufbau einer medizinischen Datenbank mit allgemeinen und Nutzerklassen-spezifischen Gesundheitsfragen;
- Figur 4:: zeigt exemplarisch einen Auszug aus der medizinischen Datenbank mit allgemeinen und Nutzerklassen-spezifischen Gesundheitsempfehlungen;
- Figur 5:: zeigt die Organisation eines Servers gemäß dem erfindungsgemäßen System zum Austausch von Gesundheitsdaten zwischen dem Server und einem Nutzer; und
- Figur 6:: zeigt ein System zum Austausch von Gesundheitsdaten zwischen einem Server und einem Nutzer sowie mit weiteren Akteuren des Gesundheitswesens.

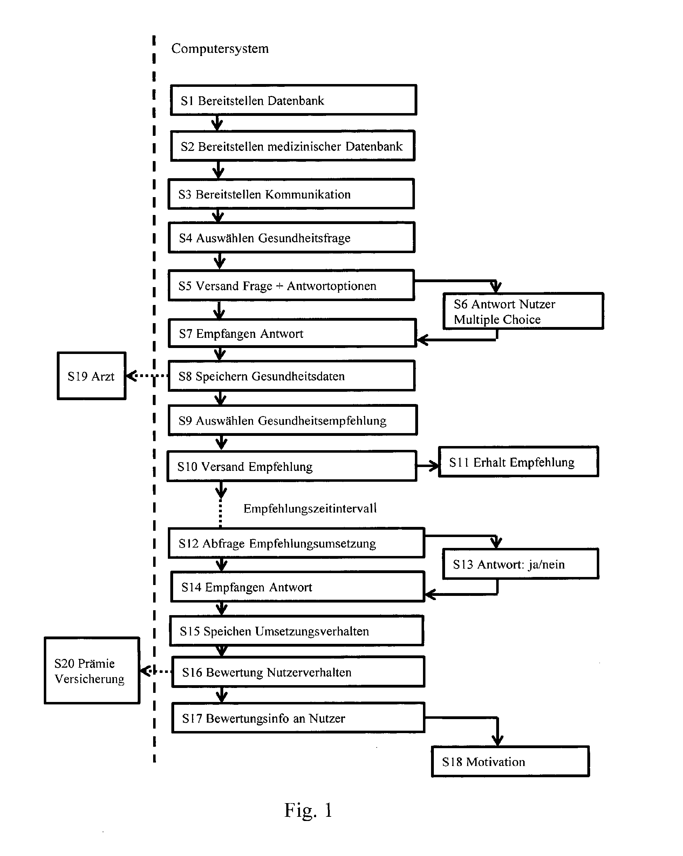

Figur 1 illustriert in Form eines Flussdiagramms das erfindungsgemäße Verfahren zum Austausch von Gesundheitsdaten zwischen einem Computersystem und einem Nutzer. Der Austausch der Gesundheitsdaten wird dabei vom Computersystem initiiert. Das bedeutet, dass die Aktion jeweils vom Computersystem ausgeht, der Nutzer reagiert (nachdem er sich einmal bei dem System angemeldet bzw. registriert hat) auf Anfragen vom Computersystem bzw. Server. Die Implementierung der Verfahrensschritte erfolgt deshalb auch auf Seiten des Computersystems, Nutzer-seitig ist lediglich die Möglichkeit zur Dateneingabe bzw. zum Datenversand an das Computersystem erforderlich. Der Nutzer benötigt also nur eine technisch sehr einfache Nutzeroberfläche, die zum Beispiel über eine einfach App für Smartphones bereitgestellt werden kann.

Beim Verfahrensschritt S1 erfolgt das Bereitstellen einer Nutzerdatenbank. In dieser Nutzerdatenbank ist Nutzerinformation betreffend Kontaktdaten des Nutzers und betreffend Gesundheitsdaten des Nutzers gespeichert. Dabei weist die Nutzerdatenbank eine besondere Struktur auf. Es ist nämlich so, dass anhand der Gesundheitsdaten eine Zuordnung jedes Nutzers zu einer bestimmten Nutzerklasse erfolgt. Im weiteren Verfahrensablauf erfolgt ein Rückgriff auf die Nutzerklasse, um die jeweilige Aktion des Systems in Hinblick auf gesundheitsfördernde Maßnahmen für einen Nutzer auszuwählen bzw. festzulegen.

Im Verfahrensschritt S2 erfolgt das Bereitstellen einer medizinischen Datenbank. Diese medizinische Datenbank weist eine besondere Struktur auf und ist eines der Herzstücke der vorliegenden Erfindung. In dieser medizinischen Datenbank befinden sich Einträge betreffend Gesundheitsfragen und betreffend Gesundheitsempfehlungen. Jeder Gesundheitsfrage ist dabei in der Datenbank eine Vielzahl von Antwortoptionen zugeordnet. Es sind also mehrere mögliche Antworten, die ein Nutzer auf diese Gesundheitsfrage hin geben kann, in der Datenbank gleich mit hinterlegt (Multiple Choice). Des Weiteren ist jede Gesundheitsfrage einer oder mehreren Nutzerklassen zugeordnet. Es ist auch möglich, dass eine Gesundheitsfrage allgemeiner Natur ist, das heißt, sie ist allen Nutzerklassen gleichermaßen zugehörig. Bei den in der Datenbank abgelegten Gesundheitsempfehlungen handelt es sich um keine Fragen, sondern um Handlungsempfehlungen für einen Nutzer, die ihn anhalten sollen, diese aktiv auszuführen und dadurch seine Gesundheit positiv zu beeinflussen. Auch die in der Datenbank hinterlegten Gesundheitsempfehlungen sind einer oder mehreren Nutzerklassen zugeordnet. Allgemeine Gesundheitsempfehlungen sind allen Nutzerklassen zugeordnet.

Im Verfahrensschritt S3 erfolgt das Bereitstellen einer Kommunikationsschnittstelle im Computersystem für Nutzer des Systems. Es wird dadurch also ganz grundsätzlich eine Kommunikation zwischen dem Computersystem und dem Nutzer ermöglicht.

Im Verfahrensschritt S4 erfolgt das Auswählen einer Gesundheitsfrage aus der medizinischen Datenbank für einen bestimmten Nutzer durch das System. Dieser Verfahrensschritt erfolgt also automatisch und wird durch das System selbst getriggert, es ist nicht vorgesehen bzw. nicht erforderlich, dass der Nutzer eine entsprechende Anfrage an das System stellt. Bei der ausgewählten Gesundheitsfrage kann es sich um eine allgemeine oder um eine im engeren Sinne Nutzerklassen-spezifische Gesundheitsfrage handeln. In jedem Fall ist sie für den speziell ausgewählten Nutzer, um den es jetzt geht, konkret geeignet.

Im nächsten Verfahrensschritt S5 erfolgt im gezeigten Beispiel ein automatisches Versenden der ausgewählten Gesundheitsfrage vom Computersystem an den Nutzer zusammen mit der zugehörigen Mehrzahl vorgegebener Antwortmöglichkeiten zu der ausgewählten Gesundheitsfrage. Im Schritt S6 antwortet der Nutzer, der die Frage erhalten hat, durch Auswahl einer Antwortmöglichkeit. In der Praxis wird beispielsweise über die entsprechende App auf einem mobilen Nutzer-Endgerät des Nutzers die Gesundheitsfrage samt Antwortoptionen dargestellt und der Nutzer wählt zum Beispiel durch eine Touch-Eingabe die auf ihn zutreffende Antwort aus. Diese wird dann an das System zurückgesendet bzw. im Verfahrensschritt S7 vom Computersystem empfangen. Es ist möglich, dass der Nutzer vor dem Versenden seiner Antwort noch einmal gebeten wird, die Korrektheit seiner Angabe zu bestätigen. Es ist aber auch möglich, dass sofort nach Eingabe der Antwort unmittelbar der Versand der Antwort an das System erfolgt.

Nachdem im Verfahrensschritt S7 die Antwort empfangen worden ist, wird im Verfahrensschritt S8 der Gesundheitszustand des Nutzers in der Nutzer-Datenbank basierend auf den erhaltenen Gesundheitsdaten gespeichert. Im beschriebenen Ausführungsbeispiel wird in der Nutzer-Datenbank also das Frage-Antwort-Protokoll der Kommunikation zwischen dem System und dem Nutzer abgelegt. Es ist auch möglich, dass nur die Daten selbst abgelegt werden oder aber gleich eine Bewertung der Daten ohne die Daten selbst (Beispiel: Blutdruck im Normbereich anstelle des konkreten Blutdruckes). Einfacher und aussagekräftiger ist aber die tatsächliche Speicherung der Gesundheitswerte selbst, bevorzugt in Kombination mit dem Zeitpunkt der Werteingabe an einem Nutzer-Endgerät bzw. dem Empfangszeitpunkt durch das System.

Im Verfahren gemäß Figur 1 wird dann in einem weiteren Verfahrensschritt S9 eine Gesundheitsempfehlung aus der medizinischen Datenbank für den ausgewählten Nutzer durch das System automatisch ausgewählt. Dabei ist es möglich, dass bei dieser Auswahl schon die Antwort des Nutzers aus Verfahrensschritt S6 mitberücksichtigt wird. Es ist aber auch möglich, dass die Gesundheitsempfehlung losgelöst von dieser Antwort automatisch durch das System ausgewählt wird. Natürlich kann die Auswahl auch schon zu einem früheren Verfahrenszeitpunkt, zum Beispiel gleichzeitig oder direkt vor oder nach dem Verfahrensschritt S4 erfolgen.

Die im Verfahrensschritt S9 ausgewählte Gesundheitsempfehlung wird dann im Verfahrensschritt S10 an den Nutzer gesendet. Dieser erhält die Gesundheitsempfehlung im Verfahrensschritt S11. Mit der in der Datenbank abgelegten Nutzerklassen-spezifischen Gesundheitsempfehlung ist ein vordefiniertes Empfehlungszeitintervall verknüpft. Dieses Intervall gibt an, welcher Zeitraum verstreichen soll, bevor das Computersystem aktiv nachfragt, ob die erhaltene Empfehlung vom Nutzer umgesetzt wurde. In der Praxis ist die Länge dieses Empfehlungszeitintervalls so bemessen, dass der Nutzer ausreichend Zeit hat, die Empfehlung umzusetzen. Das Empfehlungszeitintervall ist dabei abhängig von der jeweiligen Art der Empfehlung. Es handelt sich also um eine individuelle Zuweisung eines Empfehlungszeitintervalls zu jeder einzelnen in der Datenbank gespeicherten Gesundheitsempfehlung.

Nach dem Verstreichen des Empfehlungszeitintervalls erfolgt dann im Verfahrensschritt S12 das Versenden einer Abfrage an den Nutzer, ob die an ihn gesendete Gesundheitsempfehlung von ihm ungesetzt wurde. Bei dieser Abfrage handelt es sich im einfachsten Fall um eine Frage, die mit Ja oder Nein zu beantworten ist. Im Schritt S13 gibt der Nutzer die entsprechende Antwort. Im Schritt S14 wird die Antwort des Nutzers empfangen und im Schritt S15 automatisch vom System abgespeichert. Bei den Verfahrensschritten S8 und S15 erfolgt also fortlaufend eine Ergänzung der in der Nutzerdatenbank hinsichtlich eines Nutzers gespeicherten Gesundheitsdaten. Es entsteht eine aussagekräftige Datenhistorie.

Im optionalen Verfahrensschritt S16 erfolgt dann das Bewerten des Nutzers hinsichtlich seines Nutzungsverhaltens des Systems und insbesondere hinsichtlich seiner Ausführung von Gesundheitsempfehlungen. Dieser Schritt kann beispielsweise durch die Vergabe von Punktwerten für das Nutzerverhalten realisiert sein. Beispielsweise kann für jede Beantwortung einer Gesundheitsfrage ein Punkt vorgesehen sein und für jede Umsetzung einer Gesundheitsempfehlung ein weiterer Punkt. Es können aber auch unterschiedlich viele Punkte für das Beantworten von Gesundheitsfragen und für das Umsetzen von Gesundheitsempfehlungen vergeben werden, je nach Wertigkeit bzw. gesundheitlichem Nutzen. Wichtig bzw. vorteilhaft ist es an dieser Stelle, dass in die Bewertung des Nutzerverhaltens nicht die medizinischen Werte des Nutzers selbst eingehen, sondern dass seine Mitarbeit im Zusammenhang mit dem Austausch der Gesundheitsinformation bewertet wird. Ein Nutzer, der sehr regelmäßig die Gesundheitsfragen beantwortet und auf seine Gesundheit achtet, indem er zum Beispiel die Empfehlungen umsetzt, erfährt eine positivere Bewertung als jemand, der zwar kerngesund ist, das System jedoch praktisch nicht nutzt. Insofern werden Punkte für ein gesundheitsförderndes oder gesundheitserhaltendes Verhalten vergeben, nicht für die Gesundheit selbst. Das Verfahren ist insofern also nicht diskriminierend. Die in Schritt S16 erfolgte Bewertung oder ein zugehöriger Punktwert wird dann wiederum in der Nutzer-Datenbank abgespeichert.

Es ist möglich und auch vorteilhaft, dass in einem weiteren Verfahrensschritt S17 die erhaltene Bewertung zur Information an den Nutzer verwendet wird. Dieser erfährt dadurch im Schritt S18 eine Information und darüber hinausgehend eine mögliche Motivation. Dabei erfolgt die Motivation einerseits durch die Kenntnis der Bewertung selbst, vergleichbar mit einem Lob des Nutzers. Andererseits kann der Nutzer bei entsprechender Implementierung des erfindungsgemäßen Verfahrens auch durch einen Anbieter des Verfahrens handfeste finanzielle Prämien erhalten. Beispielsweise kann sich seine Versicherungsprämie in der Krankenversicherung reduzieren.

Bevorzugt ist es außerdem so, dass ein Nutzer des Systems die über ihn gespeicherten Daten in der Nutzer-Datenbank jederzeit komplett einsehen kann. Eine Manipulation der Daten sollte dabei aber sicher ausgeschlossen sein. Er kann sich also auch selbst Kenntnis seiner Gesundheitsdaten verschaffen, und zwar der vollständigen in der Datenbank gespeicherten Daten. Das können die Gesundheitsdaten sein, die im Rahmen der Ausführung des erfindungsgemäßen Verfahrens in der Gesundheitsdatenbank gespeichert worden sind. Weitergehend können dies aber auch die fachspezifischen Daten sein, die er zum Beispiel durch seinen Arzt hat bestimmen lassen. Es ist explizit erwünscht, dass auch Ärzte in das Verfahren entsprechend miteingebunden werden. Wenn ein Nutzer dies wünscht, ist es zum Beispiel möglich, im Rahmen eines Verfahrensschrittes S19 die aktuell ermittelten Gesundheitsdaten gleich auch an den Arzt des Nutzers zu übermitteln. Dies kann wiederum vollautomatisch geschehen. Alternativ ist es möglich, dass der Nutzer seine Daten durch den Arzt erst im Moment des nächsten Arztbesuches abruft bzw. dass die Daten dem Arzt dann erst auf konkrete Anforderung bereitgestellt werden. Es gibt viele Möglichkeiten, das erfindungsgemäße Verfahren zu diversifizieren, um dem Verfahren neue und vorteilhafte Optionen hinzuzufügen und alle Vorteile des erfindungsgemäßen Verfahrens zu nutzen.

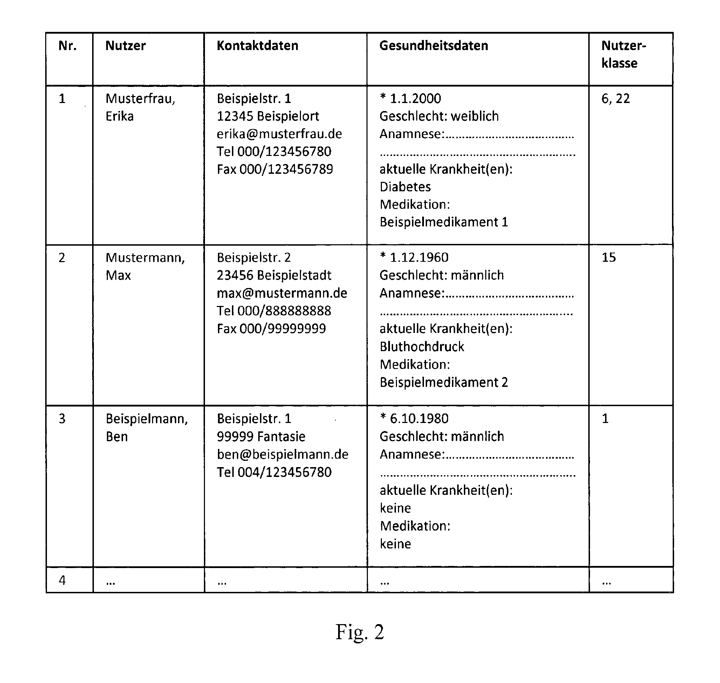

Figur 2 zeigt exemplarisch und in schematischer Darstellung einen Auszug aus einer Nutzerdatenbank. Jedem Nutzer ist im gezeigten Beispiel eine individuelle Nutzernummer zugeordnet. Erfasst sind in der Nutzerdatenbank zum Einen die Kontaktdaten des Nutzers und zum Anderen seine Gesundheitsdaten. Dem Nutzer selbst wird dabei auch mindestens eine bestimmte Nutzerklasse zugeordnet. Die Kontaktdaten beinhalten die üblichen Kontaktdaten wie Adresse, Emailadresse, Telefonnummer oder Faxnummer. Bei den Gesundheitsdaten können diverse Informationen gesundheitlicher Art über den Patienten gespeichert sein. Es wird zum Beispiel das Geburtsdatum und das Geschlecht des Nutzers erfasst. Aktuelle Krankheiten und eine aktuelle Medikation ist bei den Gesundheitsdaten gemäß Figur 2 hinterlegt. Des Weiteren kann bei den Gesundheitsdaten eine komplette Anamnese für den Nutzer hinterlegt sein. Diese Anamnese ist beliebig lang und umfassend und deshalb in Figur 2 nur durch Pünktchen angedeutet. Dem zuständigen Fachmann ist klar, wie eine solche Anamnese aufgebaut sein kann. Ganz wichtig ist in der Nutzer-Datenbank, dass dem jeweiligen Nutzer mindestens eine Nutzerklasse zugeordnet ist. Im gezeigten Beispiel handelt es sich um einfache Zahlen, die die Nutzerklasse ausdrücken. Es ist aber auch möglich, dass die Nutzerklassen eine Baumstruktur aufweisen, so dass zum Beispiel Hauptklassen bzw. Ober-Nutzerklassen und Nebenklassen bzw. Unter-Nutzerklassen verwendet werden. Dies ermöglicht zum Beispiel eine Differenzierung bzw. einen besseren Überblick bei der Verknüpfung der Nutzerklassen mit den Gesundheitsfragen und Gesundheitsempfehlungen. Es ist zum Beispiel so, dass bestimmte Gesundheitsfragen allen Nutzern mit Herz/Kreislauferkrankungen gestellt werden sollten. Diese wären dann derselben Haupt-Nutzerklasse zuzuordnen. Abhängig von der konkreten Herz/Kreislauferkrankung könnte dann eine Unter-Nutzerklasse definiert werden, wo wiederum bei den Gesundheitsfragen und Gesundheitsempfehlungen eine Differenzierung gemäß dieser Unter-Nutzerklasse erfolgen kann. Eine weitere Unterklasse kann zum Beispiel den Schweregrad einer Erkrankung oder die Medikation bei einer bestimmten Erkrankung festlegen. Auf diese Weise ist es sehr präzise möglich, jedem Nutzer des erfindungsgemäßen Verfahrens mindestens eine ganz individuell auf ihn zutreffende Nutzerklasse zuzuordnen. Dies ermöglicht eine sehr gezielte gesundheitliche Unterstützung des Nutzers. Dabei ist es auch möglich, dass in regelmäßigen Zeitabständen eine Zuordnung des Nutzers zu einer Nutzerklasse überprüft bzw. ggf. aktualisiert und/oder verfeinert wird. So ist jederzeit eine optimale Unterstützung des Nutzers basierend auf seiner Nutzerklassenzuordnung möglich.

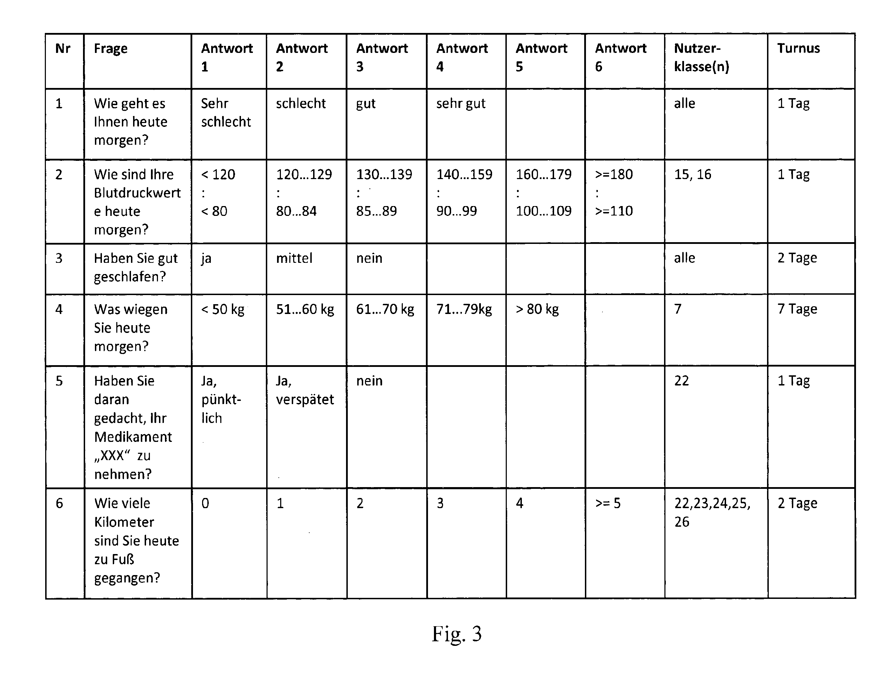

Figur 3 zeigt exemplarisch und in schematischer Darstellung einen Auszug aus der medizinischen Datenbank gemäß dem erfindungsgemäßen Verfahren. Dargestellt sind die Einträge betreffend Gesundheitsfragen in der medizinischen Datenbank. Jeder Frage ist dabei eine Kennziffer oder alternativ ein bestimmter Code zugeordnet. Neben der Frage selbst sind mehrere Antwortoptionen zu jeder Frage in der medizinischen Datenbank erfasst. Die Zahl der Antwortoptionen kann von Frage zu Frage variieren. Im gezeigten Beispiel verfügt die Frage Nummer 2 "Wie sind ihre Blutdruckwerte heute Morgen?" über 6 mögliche Antwortoptionen. Die Antworten beziehen sich dann jeweils auf bestimmte Intervalle von systolischem und diastolischem Wert. Bei diesen Antwortoptionen handelt es sich also um objektive medizinische Daten, also um Daten, die exakt und objektiv gemessen wurden. Ein Gegenbeispiel ist in Figur 3 Frage Nummer 1 "Wie geht es Ihnen heute Morgen?". Dabei handelt es sich um eine subjektive Gesundheitsfrage, die jeder Nutzer nur individuell nach seinem persönlichen Empfinden beantworten kann. Die möglichen Antwortoptionen 1 bis 4 lauten sehr schlecht, schlecht, gut und sehr gut.

Allen Gesundheitsfragen, egal ob subjektiv oder objektiv, ist mindestens eine Nutzerklasse zugeordnet. In den meisten Fällen sind einer Frage mehrere Nutzerklassen zugeordnet. Außerdem ist in der medizinischen Datenbank für jede Gesundheitsfrage ein bestimmter Turnus hinterlegt. Dieser Turnus gibt an, wie oft die Frage demselben Nutzer gestellt wird bzw. wie oft die Frage also wiederholt wird. Es entsteht dadurch eine bestimmte Messreihe mit einem bestimmten Messintervall. Im gezeigten Beispiel variiert der Frageturnus zwischen einem Tag und sieben Tagen. Der Turnus wird dabei auf die jeweilige Frage individuell angepasst. Der Frage "Was wiegen Sie heute Morgen?" ist ein Sieben-Tage-Turnus zugeordnet, da die Tendenz einer Gewichtsreduktion oder Gewichtszunahme mit einiger Aussagekraft erst nach einigen Tagen bestimmt werden kann. Tägliche Messungen sind trügerisch und verursachen häufig völlig unnötigen Stress für den Nutzer.

Es ist auch möglich, dass eine bestimmte Gesundheitsfrage noch offene Parameter bzw. Inhaltsparameter aufweist, die mit Daten eines Nutzers aus der jeweiligen Nutzerdatenbank belegt werden. Im Beispiel der Figur 3 ist Frage Nummer 5 von diesem Typ. Die Frage lautet: "Haben Sie daran gedacht, Ihr Medikament XXX zu nehmen?" Hierbei ist XXX ein Inhaltsparameter. Hier kann das System also bei der Stellung der Frage das individuelle Medikament des Nutzers aus den Infos zur Medikation in der Nutzerdatenbank mit der Gesundheitsfrage Nummer 5 verknüpfen. Je individueller ein Nutzer des erfindungsgemäßen Verfahrens bzw. Systems eine Gesundheitsfrage empfindet, desto positiver wird sein Antwortverhalten zu den Gesundheitsfragen sein. Dies wiederum bewirkt einen positiveren Effekt hin zu einer gesundheitsbewussteren Lebensweise und Lebensführung.

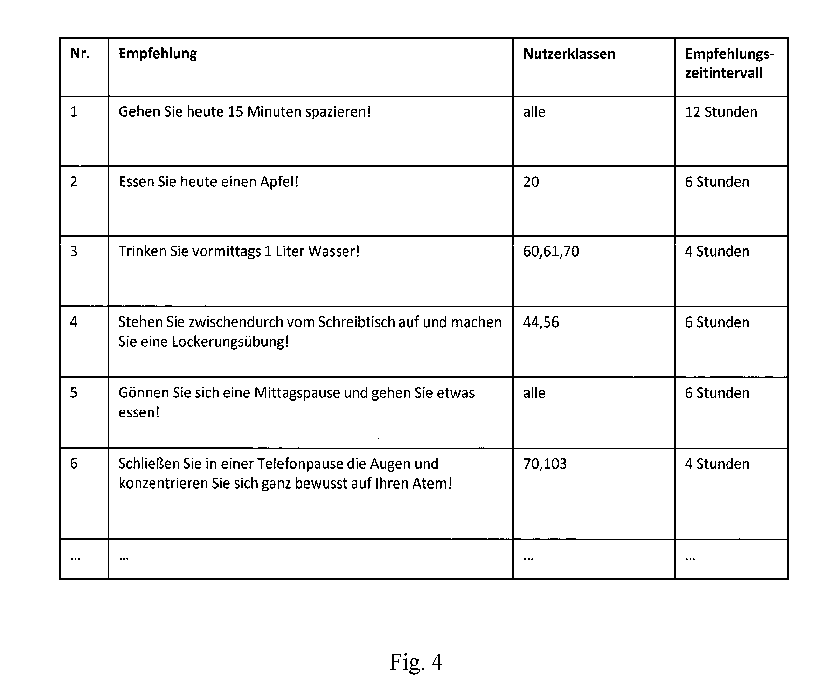

Figur 4 zeigt einen weiteren Auszug aus der medizinischen Datenbank gemäß der Erfindung, wobei in diesem Datenbank-Auszug die Einträge hinsichtlich Gesundheitsempfehlungen beispielhaft dargestellt sind. Den Gesundheitsempfehlungen ist wiederum eine Identifikationsnummer oder alternativ ein bestimmter Code zugeordnet. Jede Gesundheitsempfehlung ist einer oder mehreren Nutzerklassen zugewiesen. Es gibt also allgemeine Gesundheitsempfehlungen (Zuweisung zu allen Nutzerklassen) sowie Nutzerklassen-spezifische Gesundheitsempfehlungen. Des Weiteren ist für jede Empfehlung ein so genanntes Empfehlungszeitintervall in der Datenbank hinterlegt. Dieses Empfehlungszeitintervall wird in der Kommunikation zwischen dem Computersystem und dem Nutzer so implementiert, dass zwischen dem Versenden der Empfehlung und der Abfrage an den Nutzer, ob die Empfehlung umgesetzt wurde, genau dieses Empfehlungszeitintervall liegt. Außerdem wird bei der Feststellung des Intervalls die Zeit berücksichtigt, die typischerweise angemessen ist, damit ein Nutzer die Empfehlung auch gut umsetzen kann. Es soll durch die Empfehlung keinerlei Stressempfinden ausgelöst werden, stattdessen soll die Abfrage als sozusagen sanfte Abfrage oder gegebenenfalls als Erinnerung aufgefasst werden. Typischerweise werden die Gesundheitsempfehlungen von einem Gesundheitsexperten erarbeitet und insbesondere in Relation zu den definierten Nutzerklassen gesetzt.

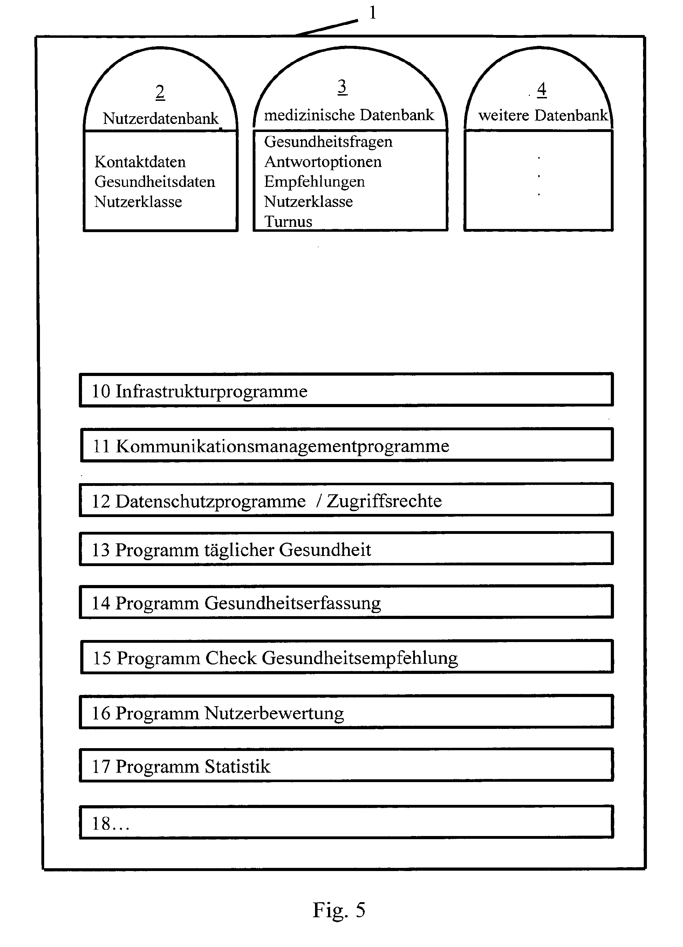

Figur 5 illustriert in schematischer Darstellung den Aufbau eines Computersystems 1 bzw. Servers 1, das zum Ausführen des erfindungsgemäßen Verfahrens geeignet ist. Der Server 1 verfügt dabei über diverse Datenbanken 2, 3, 4 etc. Datenbank 2 ist die Nutzerdatenbank 2, in der Nutzerinformationen betreffend Kontaktdaten des Nutzers und betreffend Gesundheitsdaten des Nutzers beinhaltet sind und wobei anhand der Gesundheitsdaten eine Zuordnung des Nutzers zu einer bestimmten Nutzerklasse erfolgt. In der medizinischen Datenbank 3 sind die Gesundheitsfragen mit zugehörigen Antwortoptionen, sowie die Gesundheitsempfehlungen mit ihrem Turnus hinterlegt. Sowohl für Gesundheitsfragen als auch für Gesundheitsempfehlungen erfolgt eine Zuordnung zu einer oder zu mehreren Nutzerklassen.

Die Datenbank 4 kann weitere Daten enthalten, die für das Verfahren bzw. System sinnvoll sind. Es ist beispielsweise möglich, dass in einer weiteren Datenbank 4 die Nutzerklassen-Definition selbst hinterlegt ist. Das Definieren der Nutzerklassen in einer separaten Datenbank ist insbesondere dann sinnvoll, wenn mit Haupt-Nutzerklassen und einer oder mehreren Neben-Nutzerklassen gearbeitet wird. Bei einer derartigen Baumstruktur ist dann ein besserer Überblick über die Nutzerklassen möglich und gegebenenfalls erforderliche Ergänzungen oder Änderungen können leichter in die Systemstruktur integriert werden.

Neben den Datenbanken 2, 3, 4 etc. beinhaltet der Server verschiedene Programmcodes. Diese können in Form von separaten Programmen oder in Form von verschiedenen Programmteilen eines übergeordneten Programms vorliegen. Hierzu zählen insbesondere Infrastrukturprogramme 10 für beispielsweise Servermanagement, Cloud-Anwendungen, Speicherung und Datenverarbeitung etc.

Vorgesehen sind im gezeigten Beispiel außerdem Kommunikationsmanagementprogramme 11, die eine Kommunikation bzw. einen Datenaustausch zwischen dem Server 1 und einem Dritten ermöglichen. Insbesondere werden über diese Kommunikationsmanagementprogramme auch das Versenden von Gesundheitsfragen und Gesundheitsempfehlungen sowie das Erhalten der zugehörigen Nutzerantworten abgewickelt. Bei dem vorgenannten Dritten kann es sich um einen Nutzer des Systems oder aber um einen anderen Akteur des Gesundheitswesens wie zum Beispiel einen Arzt, einen Apotheker, ein Versicherung, ein Krankenhaus etc. handeln.

Insbesondere bei einer Vielzahl von unterschiedlichen Kommunikationspartnern ist es notwendig, diverse Datenschutzprogramme und Zugriffsrechte in einem speziellen Code 12 auf dem Server 1 zu hinterlegen. So ist sichergestellt, dass nur autorisierte Akteure Zugriff auf bestimmte Daten des Systems erhalten. Diese Zugriffsrechtekönnen für verschiedene Akteure einheitlich oder aber Nutzer-individuell ja nach Einverständnis des Nutzers festgelegt werden.

Vorgesehen ist zum eigentlichen Ablauf des erfindungsgemäßen Verfahrens weiterhin ein Programmcode zur automatischen insbesondere täglichen Gesundheitsunterstützung 13. Mithilfe dieses Programmcodes werden automatisch an den Nutzer zu versendende Gesundheitsfragen inklusive zugehöriger Antwortoptionen ausgewählt. Innerhalb desselben Programmcodes kann dann auch die Auswahl von Gesundheitsempfehlungen für einen Nutzer implementiert sein. Typischerweise wird ein solcher Programmcode mehrere Teile aufweisen, mindestens einen für die Auswahl der Gesundheitsfragen und einen weiteren Programmteil für die Auswahl der Gesundheitsempfehlungen. Es ist dabei aber auch möglich, dass die Auswahl von Handlungsempfehlungen auf zuvor erhaltene Antworten des Nutzers auf eine ausgewählte Gesundheitsfrage abgestimmt wird. Es gibt also Rückkopplungsmöglichkeiten zu Nutzerverhalten bzw. zu Nutzerantworten innerhalb dieses Programmcodes.

Des Weiteren ist ein Programm 14 mit einem Programmcode zur automatischen Gesundheitszustandserfassung vorgesehen. Dabei werden insbesondere die vom Nutzer an den Server in Antwortform übermittelten Gesundheitsdaten direkt oder indirekt in der Nutzer-Datenbank 2 gespeichert.

Ein Programmcode 15 zur automatischen Ausführungskontrolle von erhaltenen Gesundheitsempfehlungen ist ebenfalls wichtiger Bestandteil des Computer-Systems 1. Es wird hierbei zum Beispiel ermittelt und gespeichert, ob und gegebenenfalls wie ein Nutzer die erhaltenen Gesundheitsempfehlungen umgesetzt hat.

Optional weist das Computersystem 1 schließlich einen Programmcode 16 zur automatischen Bewertung des Nutzers auf. Dabei werden nicht die Gesundheitswerte des Nutzers selbst, sondern das Nutzungsverhalten eines Nutzers und insbesondere die Ausführung von Gesundheitsempfehlungen durch den Nutzer bewertet. Für eine gute Mitarbeit werden zum Beispiel Punktwerte vergeben, für die auch Prämien zum Beispiel bei einem Krankenversicherer erhalten werden können.

Im gezeigten Beispiel nach Figur 5 ist des Weiteren ein Statistikprogrammcode 17 vorgesehen, der programmiert ist, um zum Beispiel die Wirksamkeit und/oder Wirtschaftlichkeit von gesundheitsfördernden Maßnahmen zu analysieren. Das Statistikprogramm kann dabei Möglichkeiten bieten, allgemeine Aussagen über die Verbesserung des Gesundheitszustandes bei bestimmten Nutzergruppen zu treffen. Es ist möglich, die Wirksamkeit bestimmter Medikamente oder mit bestimmten Medikamenten auftretende Nebenwirkungen zu untersuchen. Es ist möglich, das Nutzerverhalten bzw. die Mitarbeit von Nutzern im Rahmen der Erfindung insgesamt zu analysieren und dadurch gegebenenfalls die Implementierung der Erfindung weiter zu verbessern. Es gibt viele Möglichkeiten, eine statistische Methode zu implementieren, die interessante Rückschlüsse auf medizinische, soziologische oder wirtschaftliche Fragestellungen erlauben.

Natürlich können noch weitere Programme 18 in das Computersystem 1 integriert sein.

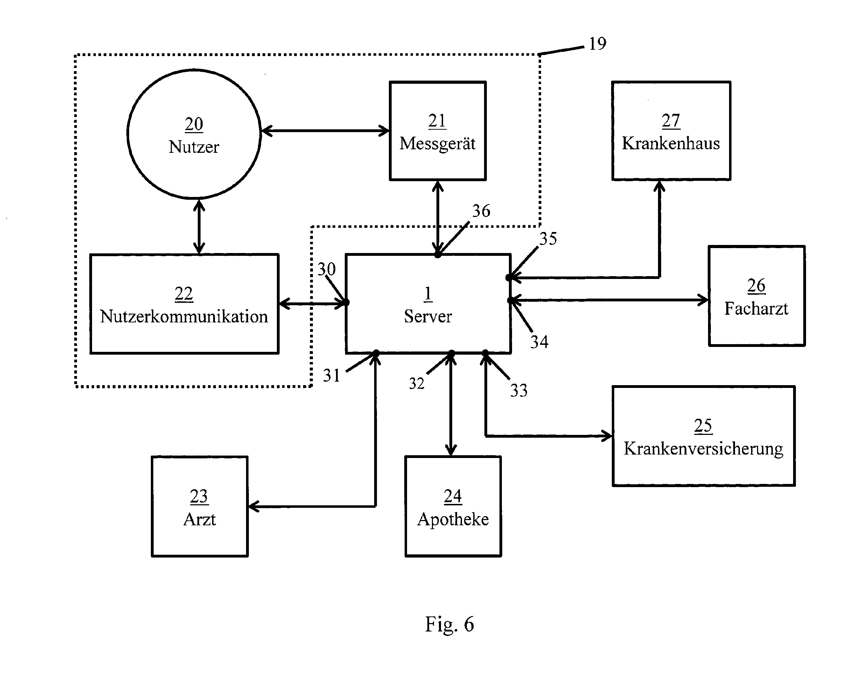

Figur 6 zeigt exemplarisch noch einmal das Computersystem bzw. den Server 1 und seine Vernetzung mit einem Nutzer 20 bzw. auch anderen Akteuren des Gesundheitssystems. Der Server 1 bildet dabei das zentrale Element des Computersystems. Der Server 1 ist mit unterschiedlichen Kommunikationsschnittstellen 30 bis 36 versehen. Über die Schnittstelle 30 erfolgt eine Kommunikation mit dem Nutzer 20. Dabei kann der Nutzer 20 ein spezielles Gerät zur Nutzerkommunikation 22, zum Beispiel ein mobiles Nutzer-Endgerät wie zum Beispiel ein Smartphone oder Tablet, verwenden. Zum Ermitteln seiner objektiven Gesundheitsdaten kann der Nutzer 20 ein Messgerät 21 verwenden. Es ist möglich, dass es sich bei diesem Messgerät 21 um ein digitales Messgerät ebenfalls mit einer Kommunikationsschnittstelle handelt. Dies erlaubt es dann, mit dem Messgerät für den Nutzer gewonnene Daten direkt auf den Server 1 und die entsprechende Datenbank 2 des Servers 1 zu überspielen. Dies ist aber nicht zwingend erforderlich. Ein Nutzer 20 kann auch mit herkömmlichen analogen Geräten Messwerte für seinen Gesundheitszustand ermitteln (zum Beispiel herkömmliche Waage oder herkömmliches Blutdruckmessgerät).

Als weitere Akteure des Gesundheitssystems sind im Beispiel gemäß Figur 6 ein Arzt 23, eine Apotheke 24, eine Krankenversicherung 25, ein Facharzt 26 und ein Krankenhaus 27 kommunikativ mit dem System 1 verknüpft. Je nach Funktion des Akteurs koppelt der Akteur an eine bestimmte Kommunikationsschnittstelle 30 bis 36 an. Es ist beispielsweise so, dass sämtliche niedergelassene Ärzte 23 über dieselbe Kommunikationsschnittstelle 31 bzw. denselben Typ von Kommunikationsschnittstelle 31 mit dem Server in Interaktion treten können. Über das Nutzen einer bestimmten Kommunikationsschnittstelle 30 bis 36 sind bestimmte Zugriffsrechte auf dem Server 1 bzw. bestimmte Datenschutzbestimmungen realisiert. Auf diese Weise können Gesundheitsdaten in dem erfindungsgemäßen System sehr zielgerichtet, umfassend, bevorzugt in Echtzeit und vollautomatisch zwischen verschiedenen Akteuren ausgetauscht werden.

Das erfindungsgemäße Verfahren, Computerprogrammprodukt und System bietet große Chancen, die Gesundheitslandschaft nachhaltig zu verbessern. Durch die Implementierung einer speziellen Kommunikationstechnologie kann erreicht werden, dass Gesundheitsdaten zum Wohle der Patienten bestmöglich ausgetauscht, ausgewertet und verwendet werden.

## Patentansprüche

1. Verfahren zum Austausch von Gesundheitsdaten zwischen einem Computersystem und einem Nutzer, das die folgenden Schritte aufweist:
Bereitstellen einer Nutzerdatenbank, wobei die Nutzerdatenbank Kontaktdaten des Nutzers und Gesundheitsdaten des Nutzers beinhaltet und wobei die Datenbank so strukturiert ist, dass anhand der Gesundheitsdaten eine Zuordnung des Nutzers zu einer Nutzerklasse erfolgt;
Bereitstellen einer medizinischen Datenbank, wobei die medizinische Datenbank Einträge betreffend Nutzerklassen-spezifische Gesundheitsfragen, eine Mehrzahl von vorgegebenen Antwortmöglichkeiten zu jeder Nutzerklassen-spezifischen Gesundheitsfrage sowie Nutzerklassen-spezifische Gesundheitsempfehlungen beinhaltet;
Bereitstellen einer Kommunikationsschnittstelle im Computersystem für Nutzer des Systems;
Auswählen einer Nutzerklassen-spezifischen Gesundheitsfrage aus der medizinischen Datenbank für einen ausgewählten Nutzer durch das System;
Auswählen einer Nutzerklassen-spezifischen Gesundheitsempfehlung aus der medizinischen Datenbank für den ausgewählten Nutzer durch das System;
Versenden der ausgewählten Gesundheitsfrage vom Computersystem an den Nutzer zusammen mit der zugehörigen Mehrzahl vorgegebener Antwortmöglichkeiten;
Empfangen einer vom Nutzer ausgewählten Antwort durch das Computersystem;
Speichern des Gesundheitszustandes des Nutzers in der Nutzerdatenbank basierend auf den in Antwortform erhaltenen Gesundheitsdaten;
Versenden der ausgewählten Gesundheitsempfehlung vom Computersystem an den Nutzer;
Versenden einer Abfrage an den Nutzer, ob die an ihn gesendete Gesundheitsempfehlung von ihm umgesetzt wurde, nach Verstreichen eines vordefinierten Empfehlungszeitintervalls;
Empfangen der Nutzerantwort zur Umsetzung der Gesundheitsempfehlung;
Speichern des Nutzerverhaltens zur Umsetzung der Gesundheitsempfehlung in der Nutzerdatenbank.

2. Verfahren gemäß dem vorangehenden Anspruch, wobei eine Nutzerklasse anhand wenigstens einem der folgenden Kriterien definiert ist: Alter des Nutzers, Geschlecht des Nutzers, Erkrankung, Schweregrad einer Erkrankung, Komplikation bei einer Erkrankung, Medikation.

3. Verfahren gemäß einem der vorangehenden Ansprüche, wobei eine Nutzerklasse durch eine Kombination aus einer Hauptklasse mit mindestens einer Nebenklasse definiert wird.

4. Verfahren gemäß einem der vorangehenden Ansprüche, das des Weiteren den folgenden Schritt aufweist:
Regelmäßiges Überprüfen der Zuordnung eines Nutzers zu einer Nutzerklasse und gegebenenfalls entsprechendes Aktualisieren seiner Nutzerklasse.

5. Verfahren gemäß einem der vorangehenden Ansprüche, wobei in der medizinischen Datenbank mindestens einer Gesundheitsfrage, insbesondere allen Gesundheitsfragen, ein Wiederholungszeitintervall als Parameter zugeordnet ist, der angibt, in welchen zeitlichen Abständen dieselbe Gesundheitsfrage an denselben Nutzer erneut versendet wird.

6. Verfahren gemäß einem der vorangehenden Ansprüche, wobei die in der medizinischen Datenbank hinterlegten Daten sowohl objektive als auch subjektive Gesundheitsdaten umfassen.

7. Verfahren gemäß dem vorangehenden Anspruch, wobei mindestens ein Gesundheitsparameter aus der folgenden Liste verwendet wird: Blutdruck, Puls, Körpergewicht, Körperfettanteil, Blutzucker.

8. Verfahren gemäß einem der vorangehenden Ansprüche, wobei ein Datensatz für eine Nutzerklassen-spezifische Gesundheitsfrage einen Inhaltsparameter aufweist, der eine individualisierende Verknüpfung mit Gesundheitsdaten eines Nutzers erlaubt.

9. Verfahren gemäß einem der vorangehenden Ansprüche, wobei das Auswählen einer Gesundheitsfrage und/oder das Auswählen einer Gesundheitsempfehlung durch das System basierend auf einem Feed-Back-Loop erfolgt.

10. Verfahren gemäß einem der vorangehenden Ansprüche, das des Weiteren den folgenden Verfahrensschritt aufweist:
Bewerten des Nutzers hinsichtlich seines Nutzungsverhaltens des Systems und insbesondere hinsichtlich seiner Ausführung von Gesundheitsempfehlungen.

11. Verfahren gemäß einem der vorangehenden Ansprüche, das des Weiteren den folgenden Verfahrensschritt aufweist:
Bereitstellen einer weiteren Kommunikationsschnittstelle für einen weiteren Akteur des Gesundheitswesens, insbesondere für einen Arzt, ein Krankenhaus, eine Apotheke, einen Krankenversicherer.

12. Verfahren gemäß einem der vorangehenden Ansprüche, das des Weiteren den folgenden Verfahrensschritt aufweist:
automatisches Austauschen von Gesundheitsdaten betreffend einen Nutzer zwischen dem Computersystem und dem weiteren Akteur des Gesundheitswesens.

13. Verfahren gemäß dem vorangehenden Anspruch, wobei das Austauschen von Gesundheitsdaten betreffend einen Nutzer zwischen dem Computersystem und mehreren Akteuren des Gesundheitswesens gleichzeitig und insbesondere in Echtzeit erfolgt.

14. Verfahren gemäß einem der vorangehenden Ansprüche, das des Weiteren den folgenden Schritt aufweist:
Analysieren der Wirksamkeit und/oder der Wirtschaftlichkeit von gesundheitsfördernden Maßnahmen.

15. Computerprogrammprodukt mit einem Programmcode zum Ausführen des Verfahrens gemäß einem der Ansprüche 1 bis 14.

16. System zum Austausch von Gesundheitsdaten zwischen einem Server und einem Nutzer, insbesondere zum Ausführen des Verfahrens gemäß einem der Ansprüche 1 bis 14,
das Folgendes aufweist:
- eine Nutzerdatenbank mit Kontaktdaten des Nutzers und mit Gesundheitsdaten des Nutzers, wobei die Datenbank so strukturiert ist, dass anhand der Gesundheitsdaten eine Zuordnung des Nutzers zu einer Nutzerklasse erfolgt;
- eine medizinische Datenbank mit Nutzerklassen-spezifischen Gesundheitsfragen und jeweils mehreren möglichen zugehörigen Antworten sowie mit Nutzerklassenspezifischen Gesundheitsempfehlungen;
- einen Programmcode zum Kommunikationsmanagement zwischen dem Server und dem Nutzer;
- einen Programmcode zur automatischen, insbesondere täglichen Gesundheitsunterstützung, der programmiert ist, um automatisch eine an den Nutzer zu versendende Nutzerklassen-spezifische Gesundheitsfrage mit zugehörigen möglichen Antwortoptionen auszuwählen, und der weiter eingerichtet ist, eine an den Nutzer zu versendende Nutzerklassen-spezifische Gesundheitsempfehlung für den Nutzer auszuwählen;
- einen Programmcode zur automatischen Gesundheitszustandserfassung des Nutzers basierend auf vom Nutzer an den Server in Antwortform übermittelten Gesundheitsdaten;
- einen Programmcode zur automatischen Ausführungskontrolle von erhaltenen Gesundheitsempfehlungen basierend auf vom Nutzer an den Server übermittelten Handlungsangaben.

17. System gemäß Anspruch 16, wobei das System eine Kommunikationsschnittstelle für mindestens einen weiteren Akteur des Gesundheitswesens aufweist, insbesondere eine Kommunikationsschnittstelle für einen Arzt, ein Krankenhaus, eine Apotheke, einen Krankenversicherer.

18. System gemäß dem vorangehenden Anspruch, wobei das System des Weiteren einen Programmcode zur automatischen Kommunikation zwischen dem Server und dem weiteren Akteur des Gesundheitswesens aufweist, wobei im Rahmen dieser Kommunikation Gesundheitsdaten betreffend einen Nutzer zwischen dem Server und dem weiteren Akteur des Gesundheitswesens ausgetauscht werden.

19. System gemäß dem vorangehenden Anspruch, wobei ein Austausch von Gesundheitsdaten betreffend einen Nutzer des Systems zwischen dem Server und mehreren Akteuren des Gesundheitssystems gleichzeitig und insbesondere in Echtzeit erfolgt.

20. System gemäß einem der vorangehenden Ansprüche 16 bis 19, wobei das System ein mobiles Nutzer-Endgerät, insbesondere ein Smartphone oder Tablet, aufweist, über das der Nutzer mit dem System kommuniziert.
